# FASCICULE DE BREVET EUROPEEN

(11) **EP 0 989 999 B1**
(45) Date de publication et mention de la délivrance du brevet: **29.09.2004**
(21) Numéro de dépôt: 98939708.8
(22) Date de dépôt: 17.07.1998
(51) Int. Cl.: C07K 14/025, C12N 15/86, A61K 39/12

(54) **COMPOSITION ANTITUMORALE A BASE DE POLYPEPTIDE IMMUNOGENE DE LOCALISATION CELLULAIRE MODIFIEE**
ANTITUMORALE ZUSAMMENSTELLUNG VON IMMUNOGENE POLYPETIDEN MIT VERÄNDERTER ZELLOKALISIERUNG
ANTITUMORAL COMPOSITION BASED ON IMMUNOGENIC POLYPEPTIDE WITH MODIFIED CELL LOCATION

(30) Priorité: 18.07.1997 FR 9709152
(43) Date de publication de la demande: 05.04.2000
(73) Titulaire: TRANSGENE S.A., 67000 Strasbourg (FR)
(72) Inventeur: KIENY, Marie-Paule, F-67100 Strasbourg (FR); BALLOUL, Jean-Marc, F-67380 Lingolsheim (FR); BIZOUARNE, Nadine, F-67300 Schiltigheim (FR)
(74) Mandataire: Warcoin, Jacques
(86) Numéro de dépôt international: PCT/FR1998/001576
(87) Numéro de publication internationale: WO 1999/003885

(56) Documents cités:
- WO-A-87/06260
- WO-A-87/07642
- WO-A-90/10459
- WO-A-93/00436
- WO-A-94/21680
- WO-A-96/39178
- WO-A-97/27216
- BOURSNELL M. ET AL.,: "Construction and characterisation of a recombinant vaccinia virus expressing human papillomavirus proteins for immunotherapy of cervical cancer" VACCINE, vol. 14, no. 16, - 1996 pages 1485-1494, XP002061738
- MENEGUZZI G ET AL: "VACCINATION AGAINST PAPILLOMAVIRUS-INDUCED TUMORS USING VACCINIA RECOMBINANTS EXPRESSING NON-STRUCTURAL PROTEINS" JOURNAL OF CELLULAR BIOCHEMISTRY, no. SUP. 13, PART C, 1 janvier 1989, page 210 XP000121130
- JARRETT W F H ET AL: "STUDIES ON VACCINATION AGAINST PAPILLOMAVIRUSES: PROPHYLACTIC AND THERAPEUTIC VACCINATION WITH RECOMBINANT STRUCTURAL PROTEINS" NUCLEIC ACIDS RESEARCH, vol. 184, 1991, pages 33-42, XP002023588
- SEEDORF K ET AL: "HUMAN PAPILLONMAVIRUS TYPE 16 DNA SEQUENCE" VIROLOGY, vol. 145, no. 1, août 1985, pages 181-185, XP002059799
- VON HEIJNE G.: 'SIGNAL SEQUENCE THE LIMIT OF VARIATION' JOURNAL OF MOLECULAR BIOLOGY vol. 184, 1985, pages 99 - 105

### LISTE DE SEQUENCES

(1) INFORMATION GENERALE:
   (i) DEPOSANT:
      (A) NOM: Transgene SA
      (B) RUE: 11 rue de Molsheim
      (C) VILLE: Strasbourg
      (E) PAYS: France
      (F) CODE POSTAL: 67082
      (G) TELEPHONE: (33) 03 88 27 91 00
      (H) TELECOPIE: (33) 03 88 27 91 11
   (ii) TITRE DE L' INVENTION: composition antitumorale a base de polypeptide immunogene de localisation cellulaire modifiée.
   (iii) NOMBRE DE SEQUENCES: 23
   (iv) FORME LISIBLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: Patentln Release #1.0, Version #1.25 (OEB)
(2) INFORMATION POUR LA SEQ ID NO: 1:
   (i) CARACTERISTIQUES DE LA SEQUENCE:
      (A) LONGUEUR: 243 acides aminés
      (B) TYPE: acide aminé
      (D) CONFIGURATION: linéaire
   (ii) TYPE DE MOLECULE: protéine
   (iii) HYPOTHETIQUE: NON
   (vi) ORIGINE:
      (A) ORGANISME: Human papillomavirus
      (B) SOUCHE: HPV-16
      (C) INDIVIDUEL ISOLE: protéine E6 fusionnee signaux de la protéine F
   (vii) SOURCE IMMEDIATE:
      (B) CLONE: E6*TMF
   (xi) DESCRIPTION DE LA SEQUENCE: SEQ ID NO: 1:
   Met Gly Leu Lys Val Asn Val Ser Ala Ile Phe Met Ala Val Leu Leu
   1               5                   10                  15
   Thr Leu Gln Thr Pro Thr Gly Gln Ile His Trp Gly Met His Gln Lys
               20                  25                  30
   Arg Thr Ala Met Phe Gln Asp Pro Gln Glu Arg Pro Arg Lys Leu Pro
           35                  40                  45
   Gln Leu Cys Thr Glu Leu Gln Thr Thr Ile His Asp Ile Ile Leu Glu
       50                  55                  60
   Cys Val Tyr Cys Lys Gln Gln Leu Leu Arg Arg Glu Val Tyr Asp Phe
   65                  70                  75                  80
   Ala Phe Arg Asp Leu Cys Ile Val Tyr Arg Asp Gly Asn Pro Tyr Ala
                   85                  90                  95
   Val Cys Asp Lys Cys Leu Lys Phe Tyr Ser Lys Ile Ser Glu Tyr Arg
               100                 105                 110
   His Tyr Cys Tyr Ser Leu Tyr Gly Thr Thr Leu Glu Gln Gln Tyr Asn
           115                 120                 125
   Lys Pro Leu Cys Asp Leu Leu Ile Arg Cys Ile Asn Cys Gln Lys Pro
       130                 135                 140
   Leu Gln Arg His Leu Asp Lys Lys Gln Arg Phe His Asn Ile Arg Gly
   145                 150                 155                 160
   Arg Trp Thr Gly Arg Cys Met Ser Cys Cys Arg Ser Ser Arg Thr Arg
                   165                 170                 175
   Arg Glu Thr Gln Leu Gly Leu Ser Ser Thr Ser Ile Val Tyr Ile Leu
               180                 185                 190
   Ile Ala Val Cys Leu Gly Gly Leu Ile Gly Ile Pro Ala Leu Ile Cys
           195                 200                 205
   Cys Cys Arg Gly Arg Cys Asn Lys Lys Gly Glu Gln Val Gly Met Ser
       210                 215                 220
   Arg Pro Gly Leu Lys Pro Asp Leu Thr Gly Thr Ser Lys Ser Tyr Val
   225                 230                 235                 240
   Arg Ser Leu
(2) INFORMATION POUR LA SEQ ID NO: 2:
   (i) CARACTERISTIQUES DE LA SEQUENCE:
      (A) LONGUEUR: 185 acides aminés
      (B) TYPE: acide aminé
      (D) CONFIGURATION: linéaire
   (ii) TYPE DE MOLECULE: protéine
   (iii) HYPOTHETIQUE: NON
   (vi) ORIGINE:
      (A) ORGANISME: human Papillomavirus
      (B) SOUCHE: HPV-16
      (C) INDIVIDUEL ISOLE: E7 fusionne signaux de la glycoproteine rabique
   (vii) SOURCE IMMEDIATE:
      (B) CLONE: E7*TMR
   (xi) DESCRIPTION DE LA SEQUENCE: SEQ ID NO: 2:
   Met Val Pro Gln Ala Leu Leu Phe Val Pro Leu Leu Val Phe Pro Leu
   1               5                   10                  15
   Cys Phe Gly Lys Phe Pro Ile Gly Ser Met His Gly Asp Thr Pro Thr
               20                  25                  30
   Leu His Glu Tyr Met Leu Asp Leu Gln Pro Glu Thr Thr Gln Leu Asn
           35                  40                  45
   Asp Ser Ser Glu Glu Glu Asp Glu Ile Asp Gly Pro Ala Gly Gln Ala
       50                  55                  60
   Glu Pro Asp Arg Ala His Tyr Asn Ile Val Thr Phe Cys Cys Lys Cys
   65                  70                  75                  80
   Asp Ser Thr Leu Arg Leu Cys Val Gln Ser Thr His Val Asp Ile Arg
                   85                  90                  95
   Thr Leu Glu Asp Leu Leu Met Gly Thr Leu Gly Ile Val Cys Pro Ile
               100                 105                 110
   Cys Ser Gln Lys Pro Arg Ser Tyr Val Leu Leu Ser Ala Gly Ala Leu
           115                 120                 125
   Thr Ala Leu Met Leu Ile Ile Phe Leu Met Thr Cys Cys Arg Arg Val
       130                 135                 140
   Asn Arg Ser Glu Pro Thr Gln His Asn Leu Arg Gly Thr Gly Arg Glu
   145                 150                 155                 160
   Val Ser Val Thr Pro Gln Ser Gly Lys Ile Ile Ser Ser Trp Glu Ser
                   165                 170                 175
   His Lys Ser Gly Gly Glu Thr Arg Leu
               180                 185
(2) INFORMATION POUR LA SEQ ID NO: 3:
   (i) CARACTERISTIQUES DE LA SEQUENCE:
      (A) LONGUEUR: 36 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Human Papillomavirus
      (B) SOUCHE: HPV-16
      (C) INDIVIDUEL ISOLE: oligonucleotide de synthese oTG5118 (E7 delete 21 26)
   (xi) DESCRIPTION DE LA SEQUENCE: SEQ ID NO: 3:
   TCTGAGCTGT CATTTAATTG AGTTGTCTCT GGTTGC 36
(2) INFORMATION POUR LA SEQ ID NO: 4:
   (i) CARACTERISTIQUES DE LA SEQUENCE:
      (A) LONGUEUR: 42 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Rabies virus
      (C) INDIVIDUEL ISOLE: oligonucleotide de mutagenese oTG5745
   (xi) DESCRIPTION DE LA SEQUENCE: SEQ ID NO: 4:
   TGCACTCAGT AATACATAGG ATCCAATAGG GAATTTCCCA AA 42
(2) INFORMATION POUR LA SEQ ID NO: 5:
   (i) CARACTERISTIQUES DE LA SEQUENCE:
      (A) LONGUEUR: 38 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (C) INDIVIDUEL ISOLE: oligonucleotide de synthese oTG6390
   (xi) DESCRIPTION DE LA SEQUENCE: SEQ ID NO: 5:
   GTATCTCCAT GCATGGATCC TGCAGGGTTT CTCTACGT 38
(2) INFORMATION POUR LA SEQ ID NO: 6:
   (i) CARACTERISTIQUES DE LA SEQUENCE:
      (A) LONGUEUR: 36 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (C) INDIVIDUEL ISOLE: oligonucleotide de synthese oTG6880
   (xi) DESCRIPTION DE LA SEQUENCE: SEQ ID NO: 6:
   GGATCCGCCA TGGTAGATCT TGGTTTCTGA GAACAG 36
(2) INFORMATION POUR LA SEQ ID NO: 7:
   (i) CARACTERISTIQUES DE LA SEQUENCE:
      (A) LONGUEUR: 32 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Rabies virus
      (B) SOUCHE: HPV-16
      (C) INDIVIDUEL ISOLE: oligonucleotide de synthese oTG5377 (E6 delete 111 a 115)
   (xi) DESCRIPTION DE LA SEQUENCE: SEQ ID NO: 7:
   TGTCCAGATG TCTTTGCAGT GGCTTTTGAC AG 32
(2) INFORMATION POUR LA SEQ ID NO: 8:
   (i) CARACTERISTIQUES DE LA SEQUENCE:
      (A) LONGUEUR: 34 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (B) SOUCHE: oligonucleotide de synthese oTG10829
   (xi) DESCRIPTION DE LA SEQUENCE: SEQ ID NO: 8:
   GCGCGCTCTA GAATTATGGG TCTCAAGGTG AACG 34
(2) INFORMATION POUR LA SEQ ID NO: 9:
   (i) CARACTERISTIQUES DE LA SEQUENCE:
      (A) LONGUEUR: 35 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (B) SOUCHE: oligonucleotide de synthese oTG10830
   (xi) DESCRIPTION DE LA SEQUENCE: SEQ ID NO: 9:
   CAGTTCTCTT TTGGTGCATG CCCCAATGGA TTTGA 35
(2) INFORMATION POUR LA SEQ ID NO: 10:
   (i) CARACTERISTIQUES DE LA SEQUENCE:
      (A) LONGUEUR: 38 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (B) SOUCHE: oligonucleotide de synthese oTG10835
   (xi) DESCRIPTION DE LA SEQUENCE: SEQ ID NO: 10:
   ATGCTAGTGC TCGATAAACC CAGCTGGGTT TCTCTACG 38
(2) INFORMATION POUR LA SEQ ID NO: 11:
   (i) CARACTERISTIQUES DE LA SEQUENCE:
      (A) LONGUEUR: 35 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (B) SOUCHE: oligonucleotide de synthese oTG10836
   (xi) DESCRIPTION DE LA SEQUENCE: SEQ ID NO: 11:
   TCAAATCCAT TGGGGCATGC ACCAAAAGAG AACTG 35
(2) INFORMATION POUR LA SEQ ID NO: 12:
   (i) CARACTERISTIQUES DE LA SEQUENCE:
      (A) LONGUEUR: 38 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (C) INDIVIDUEL ISOLE: oligonucleotide de synthese oTG10833
   (xi) DESCRIPTION DE LA SEQUENCE: SEQ ID NO: 12:
   CGTAGAGAAA CCCAGCTGGG TTTATCGAGC ACTAGCAT 38
(2) INFORMATION POUR LA SEQ ID NO: 13:
   (i) CARACTERISTIQUES DE LA SEQUENCE:
      (A) LONGUEUR: 36 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (C) INDIVIDUEL ISOLE: oligonucleotide de synthese oTG10834
   (xi) DESCRIPTION DE LA SEQUENCE: SEQ ID NO: 13:
   GCGGGCATGC GGTACCTCAG AGCGACCTTA CATAGG 36
(2) INFORMATION POUR LA SEQ ID NO: 14:
   (i) CARACTERISTIQUES DE LA SEQUENCE:
      (A) LONGUEUR: 32 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Vaccinia virus
      (B) SOUCHE: Ankara modifie
      (C) INDIVIDUEL ISOLE: oligonucleotide de synthese oTG7637 (PCR zone III)
   (xi) DESCRIPTION DE LA SEQUENCE: SEQ ID NO: 14:
   GGGGGGGAAT TCAGTAAACT TGACTAAATC TT 32
(2) INFORMATION POUR LA SEQ ID NO: 15:
   (i) CARACTERISTIQUES DE LA SEQUENCE:
      (A) LONGUEUR: 39 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Vaccinia virus
      (B) SOUCHE: Ankara modifie
      (C) INDIVIDUEL ISOLE: oligonucleotide de synthese oTG7638 (PCR zone III)
   (xi) DESCRIPTION DE LA SEQUENCE: SEQ ID NO: 15:
   GGGGGGGGAT CCGAGCTCAC CAGCCACCGA AAGAGCAAT                    39
(2) INFORMATION POUR LA SEQ ID NO: 16:
   (i) CARACTERISTIQUES DE LA SEQUENCE:
      (A) LONGUEUR: 32 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Vaccinia virus
      (B) SOUCHE: Ankara modifie
      (C) INDIVIDUEL ISOLE: oligonucleotide de synthese oTG7635 (PCR zone III)
   (xi) DESCRIPTION DE LA SEQUENCE: SEQ ID NO: 16:
   GGGGGGGGAT CCGGAAAGTT TTATAGGTAG TT 32
(2) INFORMATION POUR LA SEQ ID NO: 17:
   (i) CARACTERISTIQUES DE LA SEQUENCE:
      (A) LONGUEUR: 30 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Vaccinia virus
      (B) SOUCHE: Ankara modifie
      (C) INDIVIDUEL ISOLE: oligonucleotide de synthese oTG7636 (PCR zone III)
   (xi) DESCRIPTION DE LA SEQUENCE: SEQ ID NO: 17:
   GGGGGGGAAT TCTTTGTATT TACGTGAACG 30
(2) INFORMATION POUR LA SEQ ID NO: 18:
   (i) CARACTERISTIQUES DE LA SEQUENCE:
      (A) LONGUEUR: 77 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (B) SOUCHE: oligonucleotide de synthese oTG10502
   (xi) DESCRIPTION DE LA SEQUENCE: SEQ ID NO: 18:
   AGCTTTTTAT TCTATACTTA AAAAATGAAA ATAAACTCGA GTTGTCAAAG CATCATCTCA 60
   ACACTGACTT GAGGTAC 77
(2) INFORMATION POUR LA SEQ ID NO: 19:
   (i) CARACTERISTIQUES DE LA SEQUENCE:
      (A) LONGUEUR: 69 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (B) SOUCHE: oligonucleotide de synthese oTG10503
   (xi) DESCRIPTION DE LA SEQUENCE: SEQ ID NO: 19:
   CTCAAGTCAG TGTTGAGATG ATGCTTTGAC AACTCGAGTT TATTTTCATT TTTTAAGTAT 60
   AGAATAAAA 69
(2) INFORMATION POUR LA SEQ ID NO: 20:
   (i) CARACTERISTIQUES DE LA SEQUENCE:
      (A) LONGUEUR: 39 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (C) INDIVIDUEL ISOLE: oligonucleotide de synthese oTG5925
   (xi) DESCRIPTION DE LA SEQUENCE: SEQ ID NO: 20:
   TCAGATCTGT CGAGGGATCT GCAGCTTCTT CTAGAGGTA 39
(2) INFORMATION POUR LA SEQ ID NO: 21:
   (i) CARACTERISTIQUES DE LA SEQUENCE:
      (A) LONGUEUR: 44 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (C) INDIVIDUEL ISOLE: oligonucleotide de synthese oTG5924
   (xi) DESCRIPTION DE LA SEQUENCE: SEQ ID NO: 21:
   AGTGAATTGC TGCAGGTACC CGGATCCGCA TCGACTATCG ACAT 44
(2) INFORMATION POUR LA SEQ ID NO: 22:
   (i) CARACTERISTIQUES DE LA SEQUENCE:
      (A) LONGUEUR: 35 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Homo sapiens
      (B) SOUCHE: lignee cellulaire Daudi
      (C) INDIVIDUEL ISOLE: amorce PCR oTG6353 (clonage B7.1)
   (xi) DESCRIPTION DE LA SEQUENCE: SEQ ID NO: 22:
   TCAGCCCCTG AATTCTGCGG ACACTGTTAT ACAGG 35
(2) INFORMATION POUR LA SEQ ID NO: 23:
   (i) CARACTERISTIQUES DE LA SEQUENCE:
      (A) LONGUEUR: 33 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Homo sapiens
      (B) SOUCHE: lignée cellulaire Daudi
      (C) INDIVIDUEL ISOLE: amorce PCR oTG6352 (clonage B7.1)
   (xi) DESCRIPTION DE LA SEQUENCE: SEQ ID NO: 23:
   TTGACCCTAA AGATCTGAAG CCATGGGCCA CAC 33

## Description

La présente invention a pour objet une composition antitumorale comprenant à titre d'agent thérapeutique ou prophylactique au moins un vecteur recombinant renfermant les séquences codant pour un polypeptide immunogène modifié de manière à présenter une localisation membranaire à la surface des cellules dans lesquelles il est exprimé, différente de sa localisation native. Elle concerne également une composition antitumorale comprenant ledit polypeptide immunogène. Une telle composition est plus partiailièrement destinée au traitement ou la prévention des lésions associées aux papillomavirus.

Il est généralement admis que le cancer est une maladie qui résulte d'une perte du contrôle de la multiplication cellulaire. Ses causes peuvent être multiples et dues notamment à un dysfonctionnement de gènes cellulaires (activation par exemple par mutation somatique de gènes potentiellement oncogènes ; dérégulation de l'expression ; inhibition de l'expression de gènes suppresseurs de tumeurs) ou à l'expression indésirable de gènes viraux.

Chez l'homme, les papillomavirus (HPV) sont associés à des pathologies allant de l'infection bénigne de la peau, aux verrues et aux tumeurs malignes. Parmi les 75 types de HPV identifiés jusqu'à présent, 20 isolats distincts sont hautement spécifiques des voies génitales et 5 d'entre eux (HPV-16 et 18 et à un moindre degré les HPV-31, 33 et 45) sont clairement associés au cancer du col de l'Utérus et des voies basses. Toute une série d'études démontre le rôle transformant de ces virus, leur intégration spécifique dans le génome des cellules néoplasiques, leur activité génique dans les cellules cancéreuses et l'importance de l'expression de certains gènes viraux dans le maintien du phénotype malin des cellules néoplasiques HPV positives (Monsenego, J. Impact Medecin, 11 mars 1994).

D'une manière générale, les papillomavirus sont des virus à ADN possédant un génome circulaire d'environ 7900 paires de bases entouré par une capside protéique. Un certain nombre de types de papillomavirus, notamment bovins (BPV) et humains (HPV) ont été identifiés (Pester, 1987, in *The papovaviridae : The Papillomaviruses*, edition Salzman et Howley, Plenum Press, New York, p 1-38). Leur génome comprend une région précoce contenant les cadres de lecture E1, E2, E4, E5, E6 et E7 et une région tardive codant pour les protéines de capside L1 et L2.

Les protéines précoces ont la capacité de lier l'ADN et sont trouvées de manière prédominante dans le noyau. Les produits d'expression E1 et E2 régulent la réplication virale et l'expression des gènes viraux alors que ceux des régions E5, E6 et E7 sont impliqués dans les processus de transformation oncogénique des cellules infectées. A cet égard, il a été montré expérimentalement que la protéine E5 de BPV-1 peut *in vitro* transformer des cellules (Schlegel et al., 1986, Science *233*, 464-467). Le pouvoir transformant de E7 a été démontré pour HPV-16 et HPV-18 (Kanda et al., 1988, J. Virol. *62*, 610-613 ; Vousden et al., 1988, Oncogene Res. 3, 1-9 ; Bedell et al., 1987, J. Virol. *61*, 3635-3640) et corrélé à sa capacité à lier le produit du gène du rétinoblastome (Rb) (Munger et al., 1989, EMBO J. *8*, 4099-4105 ; Heck et al., 1992, Proc. Natl. Acad. Sci. USA *89*, 4442-4446). Par ailleurs, Crook et al. (1991, Cell *67*, 547-556) ont montré que l'antigène E6 de HPV-16 et 18 peut complexer le produit du gène p53, ce qui explique son rôle prédominant dans la transformation cellulaire.

Les pathologies associées aux virus HPV posent un problème thérapeutique du fait de leur nature persistante et récurrente. Même si les approches classiques demeurent la chirurgie et la chimiothérapie, l'immunothérapie est maintenant envisagée pour le traitement de ces maladies. Le candidat vaccin idéal doit, à titre préventif (immunoprophylaxie), empêcher l'infection de s'établir durablement et de se propager aux tissus voisins et, à titre curatif (immunothérapie), réduire l'évolution tumorale chez les patientes infectées. On a proposé jusqu'à présent d'utiliser les antigènes de capside pour induire la production d'anticorps contre les épitopes localisés à la surface des particules virales et les protéines précoces pour établir l'immunité cellulaire à l'encontre des cellules infectées après intégration de l'ADN viral.

A cet égard, le brevet européen EP 0 462 187 décnt une approche thérapeutique par administration de poxvirus exprimant les gènes précoces des papillomavirus. L'approche de vaccination décrite dans WO 93/02184 est basée sur l'utilisation des antigènes de capside à titre d'agents immunogènes et notamment de particules virales vides d'ADN (VLP pour Virus like particles) reconstituées *in vitro*. La demande française 96 09584 divulgue une composition associant l'effet préventif procuré par les polypeptides précoces et l'effet curatif conféré par les polypeptides tardifs des papillomavirus. Or, jusqu'à présent, on a mis en oeuvre les protéines virales, éventuellement mutées de manière à abolir leur activité transformante, mais néanmoins natives du point du vue de leur localisation cellulaire.

On peut également cité la demande internationale du brevet publiée sous le numéro WO 94/21680 qui décrit des vecteurs comprenant des séquences codant pour un polypeptide immunogène fusionné avec une séquence signale de réticulum endoplasmique et leur utilisation dans des compositions pour le traitement des cancers et des infections virales, la séquence signale intervenant pour permettre l'ancrage des polypeptides chimères dans la membrane du réticulum endoplasmique.

La présente invention se propose d'utiliser des protéines immunogènes dont la localisation a été modifiée dans le but d'améliorer leur accessibilité au système immun de l'hôte, afin d'améliorer ou stimuler une réponse immunitaire à l'égard de la tumeur ou du cancer à traiter, que celle-ci soit spécifique ou non spécifique et de type humoral (production d'anticorps) ou cellulaire (réponse cytotoxique CTL).

On a maintenant modifié les antigènes nucléaires E6 et E7 de HPV-16 par introduction de séquences d'ancrage et de sécrétion appropriées afin de leur conférer une présentation transmembranaire. Le changement de localisation a un effet bénéfique sur la réponse immune qui se traduit par une activité antitumorale supérieure chez les animaux traités avec un virus de la vaccine co-exprimant les antigènes E6 et E7 membranaires et l'IL-2 humaine, comparé à ceux ayant reçu un virus équivalent produisant les antigènes nucléaires. Le but de la présente invention est de mettre à la disposition du public des compositions antitumorales plus efficaces que les compositions de l'art antérieur, pour intuber au moins partiellement l'établissement ou la progression d'une tumeur ou d'un cancer. Une application particulièrement utile est le traitement des infections à HPV et plus particulièrement des pathologies graves telles que le cancer du col de l'utérus.

C'est pourquoi la présente invention a pour objet une composition antitumorale comprenant à titre d'agent thérapeutique ou prophylactique au moins un vecteur recombinant renfermant les séquences codant pour un ou plusieurs polypeptide(s) immunogène(s), l'un au moins desdits polypeptides présentant naturellement une localisation non membranaire et étant modifié par insertion d'une séquence d'ancrage membranaire et, au cas ou le polypeptide immunogène natif en est dépourvu, d'une séquence de sécrétion de manière à présenter une localisation membranaire à la surface des cellules dans lesquelles il est exprimé, ledit polypeptide immunogène présentant un degré de simalirité supérieur à 70% avec tout ou partie d'un polypeptide codé par une région précoce et/ou tardive d'un papillomavirus.

Au sens de la présente invention, le terme "polypeptide immunogène" désigne un polypeptide qui ne trouve pas son équivalent dans les cellules normales. Un exemple préféré est constitué par un antigène tumeur-spécifique. Pour illustrer, on peut citer les antigènes cellulaires dont l'expression survient pendant la période foeto-embryonnaire et régresse à la naissance jusqu'à disparaître, les antigènes qui s'expriment normalement à un très faible niveau et qui, exprimés à fort niveau, deviennent caractéristiques d'une tumeur, les antigènes cellulaires dont la structure ou la conformation est modifiée ou encore les antigènes non cellulaires, en particulier viraux dérivant d'un virus oncogène. Il peut s'agir par exemple des produits d'expression des gènes BRCA-1 (Miki et al., 1994, Science *226*, 66-71), BRCA-2 (Wooster et al., 1995, Nature *378*, 789-792), MUC-1 (Hareuveni et aL, 1990, Proc. Natl. Acad. Sci. USA *87*, 9498-9502), CEA..., dont certaines mutations ou la surexpression sont impliquées dans le développement cancéreux. Pour ce qui est des antigènes viraux, on peut citer plus particulièrement les produits d'expression des gènes précoces ou tardifs des papillomavirus, EBNA-1 du virus Epstein Barr, les antigènes des virus HTLV (Human T Lymphocyte Virus) 1 et II ou des virus de l'hépatite B et C. Les antigènes spécifiques de tumeurs sont largement décrits dans la littérature accessible à l'homme de l'art

La caractéristique essentielle du polypeptide en usage dans la présente invention est de présenter une localisation différente de sa localisation native. Les mécanismes de transport et les signaux impliqués sont décrits dans les ouvrages de biologie cellulaire (voir par exemple Molecular Biology of the Cell, Third Ed. Garland Publishing Inc. NY & London). Brièvement, la grande majorité des polypeptides est synthétisée sur des ribosomes libres dans le cytosol où ils excercent leur activité. Mais, certains polypeptides ont une destination cellulaire autre, généralement déterminée par la présence de signaux peptidiques appropriés et doivent être transportés jusqu'à celle-ci. Ainsi, les polypeptides destinés à être exportés vers la membrane plasmique ou sécrétés à l'extérieur de la cellule sont synthétisés par des ribosomes associés au réticulum endoplasmique (RE) généralement sous forme de précurseurs comportant à leur extrémité amino-terminale une séquence de sécrétion (ou peptide signal) qui initie leur passage dans le RE. Elle est ensuite éliminée par une endopeptidase spécifique pour donner le polypeptide mature. Une séquence de sécrétion comporte habituellement 15 à 35 acides aminés essentiellement hydrophobes. Il n'existe pas de séquence consensuelle et il semble que ce soit la structure secondaire qui détermine la reconnaissance par l'endopeptidase. Néanmoins, le clivage protéolytique a lieu le plus souvent après un résidu glycine, sérine ou alanine.

Les protéines membranaires comportent généralement une séquence d'ancrage de nature fortement hydrophobe qui reste insérée dans la membrane plasmique. Le polypeptide peut être transmembranaire avec l'une de ses extrémités exposée à l'extérieur de la cellule, la séquence d'ancrage traversant la membrane et l'autre extrémité du côté cytosolique. Dans la plupart des cas, la chaîne polypeptidique imbriquée dans la double couche lipidique de la membrane a une conformation en hélice α (voir par exemple Branden et Tooze, 1991, dans Introduction to Protein Structure p 202-214, NY Garland).

Quant à la localisation nucléaire, elle peut être conférée par la présence d'une courte séquence dite de localisation nucléaire (NLS) composée principalement de résidus chargés positivement, tels que lysine et arginine. On peut citer à titre d'exemples les signaux de translocation nucléaire KRKKRK et RKRRKR présents dans les polypeptides L1 et L2 de HPV (Zhou et al., 1991, Virology *185*, 625-632). Cependant, certains polypeptides excerçant leur fonction au sein du noyau ne possèdent pas de séquence NLS typique. C'est le cas des antigènes E6 et E7 de papillomavirus.

Le polypeptide immunogène compris dans la composition selon l'invention peut résulter de l'introduction et/ou la délétion de signaux de localisation appropriés au sein d'un polypeptide natif, d'un fragment de celui-ci, d'une chimère comportant des séquences d'origines différentes ou d'un variant (délétion, insertion et/ou substitution d'un ou plusieurs acides aminés). De manière plus particulière, sa séquence en acides aminés présente un degré de similarité avec tout ou partie de la séquence du polypeptide natif dont il est issu supérieur à 70 %, avantageusement, supérieur à 80 % et, de préférence, supérieur à 90 %. Le degré de similarité peut être aisément calculé à l'aide d'un programme ordinateur approprié ou en alignant les séquences de manière à obtenir le degré maximal d'homologie et en comptabilisant le nombre de positions dans lesquelles les acides aminés des deux séquences se retrouvent à l'identique par rapport au nombre total de positions.

L'homme du métier connait les signaux permettant le changement de présentation cellulaire d'un polypeptide. Dans le cas où l'on désire que le polypeptide immunogène soit sécrété, l'adjonction d'une séquence de sécretion à son extrémité amino-terminale, permettra son transport via le RE vers l'extérieur de la cellule hôte. L'insertion a lieu de préférence immédiatement en aval du codon initiateur de la traduction. Dans le cadre de la présente invention, il peut être avantageux de muter/déléter tout ou partie des résidus déterminant la localisation native, pour éviter les interférences. Par exemple, si le polypeptide natif a une destination membranaire, il comporte d'ores et déjà une séquence de sécretion et on procédera éventuellement à la mutation ou délétion de la séquence d'ancrage hydrophobe. En outre, l'inactivation (par mutation/délétion) des signaux natifs peut permettre une localisation cytoplasmique. La présentation cytoplasmique d'un peptide immunogène présentant normalement une localisation cellulaire différente (par exemple nucléaire, membranaire, sécrétée...) peut favoriser la présentation peptidique médiée par les antigènes de classe 1 du complexe majeur d'histocompatibilité et la réponse CTL *in vivo* (Tobery et Siliciano, 1997, J. Exp. Med. *5*, 909-920). Un autre mode de réalisation envisageable consiste à fusionner le polypeptide immunogène avec l'ubiquitine également dans le but de stimuler une réponse CTL puissante.

La composition selon l'invention comprend au moins un vecteur recombinant renfermant les séquences codant pour un polypeptide immunogène modifié de manière à présenter une localisation membranaire à la surface des cellules dans lesquelles il est exprimé, par insertion d'une séquence d'ancrage membranaire et, dans le cas où le polypeptide natif en est dépourvu, d'une séquence de sécrétion. Le site d'insertion préféré de la séquence de sécrétion est l'extrémité N-terminale, comme indiqué précédemment, et celui de la séquence d'ancrage membranaire est l'extrémité C-terminale, par exemple immédiatement en amont du codon stop. Dans ce contexte, il peut également être avantageux de procéder à la mutation/détetion de tout ou partie des signaux de localisation natifs (par exemple séquence NLS) pour ne pas interférer avec la nouvelle localisation.

Le choix du signal de localisation susceptible d'être mis en oeuvre dans le cadre de la présente invention est vaste. Il peut dériver de toute protéine en comportant d'origine eucaryote ou non (virus, parasite, champignon ....) du moment qu'il soit reconnu par la cellule à traiter. Il peut être naturel ou synthétique, hétérologue ou homologue vis à vis de cette dernière. Il peut également comporter une ou plusieurs modifications par rapport au signal dont il dérive, sous réserve qu'elle(s) n'affecte(nt) pas sa fonction. A titre indicatif, on préférera avoir recours aux séquences de sécrétion et/ou d'ancrage membranaire de la glycoprotéine rabique, de la glycoprotéine env du virus HIV ou de la protéine F du virus de la rougeole. Dans le cas où la modification du polypeptide immunogène fait intervenir plusieurs signaux de localisation (par exemple séquence de sécrétion et d'ancrage membranaire), ceux-ci peuvent avoir une origine commune ou différente.

Il est également possible de mettre en oeuvre des signaux de localisation ciblant un compartiment cellulaire particulier. On peut citer notamment une séquence consensus d'endocytose (par exemple celle présente dans la région C-terminale des chaînes lourdes d'immunoglobuline IgG1 de séquence IPNYRNM ; Kaisho et al., 1997, Science 276, 412-414) ou une séquence permettant le ciblage dans la membrane de l'appareil de Golgi (Mochamer et Rose, 1987, J. Cell Biol. *105*, 1205-1214 ; Mochamer, 1993, Curr. Opin. Cell Biol. *5*, 606-612). On peut notamment envisager l'emploi de séquences dérivées de la glycoprotéine E1 de Coronavirus divulguées dans la banque de données Swiss-Prot sous, l'accession P11222. Ce type de séquence peut être incorporé à l'extrémité C-terminale du polypeptide immunogène.

Par ailleurs, la modification de la localisation cellulaire peut être réalisée par toute technique conventionnelle, notamment par mutagénèse dirigée, ligation de signaux exogènes ou PCR.

Selon un mode de réalisation préféré, une composition antitumorale selon l'invention, est destinée à traiter ou prévenir les infections à papillomavirus et les désordres en résultant, en particulier les dysplasies du col de bas grade et le cancer du col de l'utérus. La composition antitumorale selon l'invention comprend au moins un polypeptide immunogène originaire d'une région précoce et/ou tardive d'un papillomavirus , notamment d'un virus à risque tel que HPV-16, 18, 31, 33 ou encore 45.

Conformément aux buts poursuivis par la présente invention, on peut mettre en oeuvre un ou plusieurs polypeptides immunogènes de papillomavirus quels qu'ils soient. Comme rappelé précédemment, leur génome code pour 8 polypeptides, deux polypeptides tardifs L1 et L2 composant la capside virale et 6 polypeptides précoces (E1, E2, E4, E5, E6 et E7) impliqués dans la régulation, le maintien du génome viral et la transformation des cellules infectées.

S'agissant d'un polypeptide immunogène de type précoce, on choisit avantageusement de mettre en oeuvre un polypeptide E6 ou E7, modifié notamment de manière à présenter une localisation membranaire. Etant donné les observations rappelées plus haut sur le pouvoir transformant, on a de préférence recours à un variant non oncogène muté au niveau de la région impliquée dans le processus de transformation cellulaire. De tels variants sont décrits dans la littérature (Munger et al., 1989, EMBO J. *8*, 4099-4105 ; Crook et al., 1991, Cell *67*, 547-556 ; Heck et al., 1992, Proc. Natl. Acad. Sci. USA *89*, 4442-4446 ; Phelps et al., 1992, J. Virol. *66*, 2418-2427). Un polypeptide immunogène convenant particulièrement aux fins de la présente invention, est l'antigène E6 de HPV-16 délété des résidus 111 à 115 (+1, représentant le premier acide aminé de l'antigène viral natif) et fusionné aux signaux de sécrétion et d'ancrage de la protéine F du virus de la rougeole (SEQ ID NO: 1). On peut également utiliser l'antigène E7 de HPV-16 délété des résidus 22 à 25 et fusionné aux séquences d'ancrage et de sécrétion de la glycoprotéine rabique (SEQ ID NO: 2).

La composition antitumorale selon l'invention peut également comprendre un polypeptide immunogène originaire de la région tardive d'un papillomavirus, dérivant de L1 ou L2.

Bien entendu, la composition antitumorale selon l'invention peut comprendre plusieurs polypeptides immunogènes, dont l'un au moins présente une localisation cellulaire différente de sa localisation native. On peut citer pour illustrer une composition associant plusieurs polypeptides dérivés de papillomavirus, ceux-ci pouvant avoir une origine commune ou différente (par exemple HPV-16 et 18 dans le but d'élargir le spectre d'action). Une composition combinant plusieurs polypeptides d'origine précoce permettra d'améliorer l'effet thérapeutique. La combinaison des polypeptides dérivés de L1 et L2 pourrait avoir un effet bénéfique sur les propriétés préventives de la composition. Enfin, une composition qui convient tout particulièrement aux buts poursuivis par la présente invention comprend au moins un polypeptide précoce et au moins un polypeptide tardif de papillomavirus afin de combiner l'effet préventif et curatif Selon un mode préféré, l'un au moins des polypeptides immunogènes d'origine précoce est modifié de manière à présenter une localisation membranaire par adjonction de séquences de sécrétion et d'ancrage telles que celles citées auparavant.

A cet égard, une composition préférée selon l'invention comprend :
(1) un polypeptide immunogène ayant une séquence homologue ou identique à celle montrée dans la SEQ ID NO: 1,
(2) un polypeptide immunogène ayant une séquence homologue ou identique à celle montrée dans la SEQ ID NO: 2,
(3) un polypeptide immunogène ayant une séquence homologue ou identique à celle montrée dans la SEQ ID NO: 1 et un polypeptide immunogène ayant une séquence homologue ou identique à celle montrée dans la SEQ ID NO: 2,
(4) un polypeptide immunogène ayant une séquence homologue ou identique à celle montrée dans la SEQ ID NO: 1, un polypeptide immunogène étant la protéine L1 d'un papillomavirus et/ou un polypeptide immunogène étant la protéine L2 d'un papillomavirus,
(5) un polypeptide immunogène ayant une séquence homologue ou identique à celle montrée dans la SEQ ID NO: 2, un polypeptide immunogène étant la protéine L1 d'un papillomavirus et/ou un polypeptide immunogène étant la protéine L2 d'un papillomavirus, ou
(6) un polypeptide immunogène ayant une séquence homologue ou identique à celle montrée dans la SEQ ID NO: 1, un polypeptide immunogène ayant une séquence homologue ou identique à celle montrée dans la SEQ ID NO: 2, un polypeptide immunogène étant la protéine L1 d'un papillomavirus et/ou un polypeptide immunogène étant la protéine L2 d'un papillomavirus.

Le terme «homologue» fait référence à un degré d'identité avec ladite séquence supérieure à 70 %, avantageusement supérieur à 80 %, de préférence, supérieure à 90 % et, de manière tout à fait préférée, supérieur à 95 %.

Avantageusement, une composition antitumorale selon l'invention peut comprendre en outre au moins un composé améliorant son effet antitumoral, dans le but d'augmenter l'intensité de la réponse immunitaire spécifiquement ou non. Outre les adjuvants, les immunostimulateurs représentent des composés particulièrement préférés. Par "immunostimulateur", on entend un composé ayant la capacité de renforcer une réponse immunitaire humorale afin d'amplifier la production d'anticorps dirigés contre le polypeptide immunogène ou à médiation cellulaire, afin de déclencher une réponse cytotoxique significative à l'encontre des cellules tumorales ou infectées. A titre indicatif, l'immunostimulation peut être évaluée en modèle animal cancéreux par comparaison du taux de rejet dans un animal traité avec le polypeptide immunogène, ceci en présence et en absence de l'immunostimulateur. D'une manière plus générale, les moyens pour mettre en évidence une inununostimulation sont indiqués dans Roitt (*Immunology*, 4th edition, Moby Ltd). Un des avantages d'une telle composition est qu'elle combine l'immunité spécifique induite par le polypeptide immunogène et l'immunité aspécinque induite par la molécule immunostimulatrice.

Dans le cadre de la présente invention, on peut utiliser un immunostimulateur natif, notamment d'origine humaine, une partie de celui-ci, une chimère provenant de la fusion de séquences d'origines diverses ou encore un mutant, à la condition toutefois de conserver la fonction immunostimulatrice. Parmi toutes les molécules envisageables, on préférera mettre en oeuvre un immunostimulateur choisi parmi l'interleukine-2, l'interleukine-7, l'interleukine-12 et les molécules de co-adhésion B7.1 et B7.2. On indique que l'interleukine-2 et la molécule B7.1 sont particulièrement préférées.

D'une manière générale, les polypeptides immunogènes et immunostimulateurs peuvent être produits par les méthodes conventionnelles de synthèse chimique ou bien par les techniques de l'ADN recombinant (voir par exemple Maniatis et al., 1989, *Laboratory Manual*, Cold Spring Harbor, Laboratory Press, Cold Spring Harbor, NY). Plus particulièrement un procédé de préparation comprend l'acte de cultiver une cellule transformée par un fragment d'ADN codant pour le polypeptide en question pour générer une cellule productrice et l'acte de récolter ledit polypeptide à partir de la culture. La cellule productrice peut être d'une origine quelconque et sans limitation, une bactérie, une levure ou bien une cellule de mammifère, dans la mesure où le fragment d'ADN considéré est soit intégré dans son génome soit intégré dans un vecteur d'expression approprié. Bien entendu, le fragment d'ADN est placé sous le contrôle de signaux de transcription et de traduction permettant son expression dans la cellule productrice. Vecteurs d'expression et signaux de contrôle sont connus de l'homme du métier.

La présente invention vise ainsi une composition antitumorale comprenant à titre d'agent thérapeutique ou prophylactique au moins un vecteur recombinant comprenant les séquences codant pour un ou plusieurs polypeptides immunogènes selon la présente invention et, éventuellement, pour un composé améliorant l'effet antitumoral. Ce type de composition présente l'avantage d'une production bon marché et d'une grande stabilité dans des conditions d'environnement variées. En particulier, les conditions de conservation sont moins contraignantes. Les polypeptides ont les caractéristiques telles que définies ci-avant.

Les séquences codant pour le polypeptide immunogène ou améliorant l'effet antitumoral peuvent être obtenues par clonage, par PCR (Polymerase Chain Réaction) ou par synthèse chimique selon les techniques conventionnelles communément en usage et à partir des données de la littérature. Pour ce qui est du mode de réalisation préféré, les séquences codant pour les polypeptides de papillomavirus peuvent être isolées à partir de cellules papillomavirus positives obtenues de patients ou de collections. L'insertion des signaux de localisation appropriés peut être réalisée par les techniques de biologie moléculaire. Les séquences codant pour l'immunostimulateur peuvent être donées à partir de l'ADN cellulaire ou des ARN messagers d'une cellule dans laquelle il est exprimé. L'homme du métier est capable de générer les sondes ou amorces appropriées à partir des données publiées. On indique que la séquence nucléotidique des génomes HPV-16 et 18 est divulguée dans Genbank aux numéros d'accession K02718 et X05015 respectivement. La séquence du gène humain IL-2 est décrite dans le brevet français 85 09480 et dans Taniguchi et aL (1983, Nature 302, 305-311) et celle codant pour l'antigène B7.1 dans Freeman et al. (1989, J. of Immunology *143*, 2714-2722).

Un vecteur préféré dans le cadre de l'invention peut être un poxvirus. Un vecteur plasmidique ou viral, notamment un adénovirus, un rétrovirus, un virus de l'herpès ou un virus associé à l'adénovirus peut être utilisé. Avantageusement, il s'agira d'un vecteur non-intégratif et d'une virulence atténuée. De tels vecteurs ainsi que leurs techniques de préparation sont connus de l'homme de l'art.

Dans le cas où l'on met en oeuvre un vecteur adénoviral, on aura de préférence recours à un vecteur non replicatif par délétion de régions essentielles à la replication et, notamment, de la majorité de la région E1 afin d'éviter sa propagation au sein de l'organisme hôte ou l'environnement. Il va de soi que l'on peut modifier ou déléter d'autres régions du génome adénoviral, notamment au sein des régions E2, E4 et/ou L1-L5, dans la mesure où les fonctions essentielles défectives sont complémentées en *trans.* Pour illustrer ces modes de réalisation, on peut citer la mutation thermosensible affectant le gène DBP (pour DNA Binding Protein en anglais) de la région E2A (Ensinger et al., 1972, J. Virol. *10*, 328-339). Une délétion partielle de la région E4 à l'exception des séquences codant pour les cadres de lecture ouverts (ORF) 6 et 7 est également envisageable (Ketner et al., 1989, Nucleic Acids Res. *17*, 3037-3048). Une autre possibilité est la délétion totale de l'unité transcriptionnelle E4. Par ailleurs, le vecteur adénoviral selon l'invention peut être dépourvu de tout ou partie de la région non essentielle E3. Selon cette alternative, il peut être intéressant de conserver néanmoins les séquences E3 codant pour les polypeptides permettant l'échappement au système immunitaire de l'hôte, notamment la glycoprotéine gp19k (Gooding et al., 1990, Critical Review of Immunology *10*, 53-71). Un vecteur adénoviral préféré selon l'invention retiendra au minimum les séquences essentielles à l'encapsidation, à savoir les ITRs (Inverted Terminal Repeat) 5' et 3' et la région d'encapsidation. On indique qu'il peut dériver d'un adénovirus humain ou animal et d'un sérotype quelconque. Les adénovirus humains du sous groupe C et notamment les adénovirus 2 (Ad2) et 5 (Ad5) conviennent tout particulièrement à la mise en oeuvre de l'invention. Les différents vecteurs adénoviraux ainsi que leurs techniques de préparation sont conventionnels et sont décrits dans Graham et Prevect (1991, in *Methods in Molecular Biology*, vol 7, p 109-128 ; Ed : E.J. Murey, The Human Press Inc.) et dans la demande internationale WO 94/28152. Par exemple, il peut être généré *in vitro* dans *Escherichia coli* (*E.coli*) par ligation ou recombinaison homologue (voir par exemple la demande internationale W096/17070) ou encore par recombinaison dans une lignée de complémentation.

S'il s'agit d'un rétrovirus, on conserve les LTRs (Long Terminal Repeat) et les séquences d'encapsidation (voir par exemple Naviaux et Verma, 1992, Current Opinion in Biotechnology 3, 540-547). La séquence codant pour le(s) potypeptide(s) immunogène(s) peut être placée sous le contrôle du LTR rétroviral ou d'un promoteur interne tels que ceux décrits ci-après. Il peut dériver d'un rétrovirus d'une origine quelconque (murin, primate, félin, humain etc...) et en particulier du MoMuLV (Moloney murine leukemia virus), MVS (Murine sarcoma virus) ou Friend murine retrovirus (Fb29). Il peut également comporter des modifications notamment au niveau des LTR (remplacement de la région promotrice par un promoteur eucaryote) ou de la région d'encapsidation (remplacement par une région d'encapsidation hétérologue, par exemple de type VL30) (voir les demandes françaises 94 08300 et 97 05203).

Selon un mode de réalisation avantageux, un vecteur recombinant selon l'invention est un poxvirus et, notamment, d'un poxvirus aviaire, tel que le poxvirus du canari, d'un fowlpox ou d'un virus de la vaccine, ce dernier étant préféré. Parmi tous les virus de la vaccine envisageables dans le cadre de la présente invention, on choisit de préférence les souches Copenhague, Wyeth et Ankara modifiée (MVA pour Modified Vaccinia Virus Ankara).

Généralement, le site d'insertion est choisi dans une région non essentielle à la réplication de sorte que les capacités de réplication et propagation du virus recombinant ne sont pas altérées. A titre indicatif, lorsque l'on utilise un virus de la souche Copenhague, le site d'insertion préféré est le locus TK, ce qui a pour effet d'inactiver celui-ci et ainsi faciliter la sélection des recombinants. On peut également utiliser le locus K1L. Pour ce qui est d'un virus MVA, l'insertion des séquences recombinantes (immunogènes et immunostimulatrices) peut être réalisée au sein de l'une au moins des excisions I à VI et, notamment II ou III (Meyer et al., 1991, J. Gen. Virol. *72*, 1031-1038 ; Sutter et al., 1994, Vaccine *12*, 1032-1040). L'insertion peut également avoir lieu dans une région virale essentielle, comme la région D4R, la fonction défective pouvant être fournie *en trans* par exemple par le biais d'une lignée de complémentation.

Bien entendu, dans le cadre de la présente invention, les séquences codant pour le polypeptide immunogène ou améliorant l'effet antitumoral sont placées sous le contrôle des éléments nécessaires à leur expression dans une cellule ou un organisme hôte. Ceux-ci incluent les éléments de régulation de la transcription ainsi que des signaux d'initiation et de terminaison de la traduction. Parmi eux, le promoteur revêt une importance particulière. D'une façon générale, on aura recours à un promoteur fonctionnel dans l'organisme ou la cellule hôte que l'on veut traiter et adapté au vecteur employé. En outre, il peut être modifié de manière à contenir des séquences régulatrices, par exemple un élément activateur de la transcription ou des séquences répondant à certains signaux cellulaires. A cet égard, il peut être avantageux d'utiliser un promoteur tissu-spécifique puisque les lésions associées aux papillomavirus sont localisées au niveau des voies génitales ou un promoteur répondant à des signaux spécifiquement tumoraux (par exemple activé en présence de facteurs de croissance généralement surexprimés par les cellules tumorales) afin de limiter l'expression aux seules cellules tumorales.

Parmi les promoteurs envisageables dans le cadre de l'invention, on peut citer les promoteurs SV40 (Virus Simian 40), HMG (Hydroxy-Methyl-Glutaryl-coenzyme A), TK (Thymidine Kinase), CMV (cytomégalovirus), RSV (Rous Sarcoma Virus), MLP (Major Late Promoter) adapté au vecteur adénoviraux et le LTR du Mo-MLV (Moloney Murine Leukemia Virus) plus spécifique des vecteurs rétroviraux. Le promoteur précoce du Cytomégalovirus (CMV) est tout particulièrement préféré. Il peut également s'agir d'un promoteur stimulant l'expression dans une cellule tumorale ou cancéreuse. On peut citer notamment les promoteurs des gènes MUC-1 surexprimé dans les cancers du sein et de la prostate (Chen et al., 1995, J. Clin. Invest. *96*, 2775-2782), tyrosinose surexprimé dans les mélanomes (Vile et al., 1993, Cancer Res. *53*, 3860-3864) et ERB-2 surexprimé dans les cancers du sein et du pancréas (Harris et al., 1994, Gene Therapy *1*, 170-175). Les promoteurs en question sont décrits dans la littérature et peuvent être clonés à partir du génome cellulaire ou viral par les techniques classiques.

S'agissant d'un vecteur poxviral, on aura recours à un promoteur pox, par exemple 7,5K, H5R, TK, p.28, p.11 ou encore K1L du virus de la vaccine. Un promoteur synthétique convient également à la mise en oeuvre de la présente invention (voir par exemple Chakrabarti et al., 1997, Biotechniques *23*, 1094-1097 ; Hammond et al., 1997, J. Virological Methods *66*, 135-138 et Kumar et Boyle, 1990, Virology *179*, 151-158). A cet égard, il s'agit avantageusement d'un promoteur chimère entre un promoteur tardif et un promoteur précoce.

Par ailleurs, les éléments nécessaires à l'expression peuvent également comporter des séquences améliorant l'expression ou le maintien dans la cellule hôte (intron, séquence terminatrice de la transcription, site d'initiation de la traduction....). Cependant, dans le cas d'un vecteur poxviral, on évitera l'emploi d'introns.

Une composition selon l'invention peut comprendre un seul ou plusieurs vecteurs recombinants exprimant les séquences correspondant aux polypeptides choisis placés, sous le contrôle d'éléments indépendants ou communs. Selon cette dernière option, on peut avoir recours à des séquences permettant d'initier la traduction de manière interne (IRES) ou à des fusions en phase des différents gènes.

Les conditions générales d'obtention d'un vecteur recombinant en usage dans la présente invention sont largement décrites dans l'état de la technique. S'agissant d'un vecteur poxviral, on peut se référer au brevet européen EP 83 286 dont le contenu est ici incorporé par référence. Ces conditions sont applicables aux autres virus acceptables comme vecteur qui possèdent une région génomique dans laquelle les blocs d'expression peuvent être incorporés. Bien entendu, ils peuvent être insérés dans le même locus ou un locus différent.

Conformément aux buts poursuivis par la présente invention, un vecteur recombinant peut en outre comprendre un bloc d'expression d'un gène marqueur de sélection afin de faciliter les étapes d'isolement et de purification du virus recombinant. On peut citer notamment le gène *neo* conférant la résistance à l'antibiotique G418, le géne *pac* de résistance à la puromycine, le gène TK du virus de l'herpès simplex de type 1 (HSV-1) qui confère la sensibilité à certains analogues de nucléosides tels que le ganciclovir ou l'acyclovir, le gène *gpt* (xanthine guanine phosphoribosyl transférase), les gènes bactériens *LacZ* codant pour la β-galactosidase et *gus A* codant pour la β-glucuronidase. Ces deux derniers marqueurs enzymatiques permettent de repérer les virus recombinants par coloration en présence des substrats X-Gal (5-bromo-4-chloro-3-indolyl-β-D-galactopyranoside) et XglcA (5-bromo-6-chloro-3-indolyl-β-D-glucoronide) respectivement.

Une composition antitumorale préférée est destinée au traitement ou la prévention d'une infection ou tumeur à papillomavirus, et comprend au moins un virus de la vaccine de la souche Copenhague ou MVA dans lequel sont insérées :
(1) les séquences codant pour un polypeptide immunagène ayant une séquence homologue ou identique à celle montrée dans la SEQ ID NO: 1,
(2) les séquences codant pour un polypeptide immunogène ayant une séquence homologue ou identique à celle montrée dans la SEQ ID NO: 2,
(3) les séquences codant pour un polypeptide immunogène ayant une séquence homologue ou identique à celle montrée dans la SEQ ID NO: 1 et pour un polypeptide immunogène ayant une séquence homologue ou identique à celle montrée dans la SEQ ID NO: 2,
(4) les séquences codant pour un polypeptide immunogène ayant une séquence homologue ou identique à celle montrée dans la SEQ ID NO: 1, pour un polypeptide immunogène étant la protéine L1 d'un papillomavirus et/ou pour un polypeptide immunogène étant la protéine L2 d'un papillomavirus,
(5) les séquences codant pour un polypeptide immunogène ayant une séquence homologue ou identique à celle montrée dans la SEQ ID NO: 2, pour un polypeptide immunogène étant la protéine L1 d'un papillomavirus et/ou un pour polypeptide immunogène étant la protéine L2 d'un papillomavirus, ou
(6) les séquences codant pour un polypeptide immunogène ayant une séquence homologue ou identique à celle montrée dans la SEQ ID NO: 1. pour un polypeptide immunogène ayant une séquence homologue ou identique à celle montrée dans la SEQ ID NO: 2, pour un polypeptide immunogène étant la protéine L1 d'un papillomavirus et/ou pour un polypeptide immunogène étant la protéine L2 d'un papillomavirus.

Ladite composition peut également comprendre les séquences codant pour un immunostimulateur de préférence choisi parmi LTL-2 ou B7.1. Il peut être porté par l'un des vecteurs recombinants permettant l'expression du ou des gènes immunogènes ou par un vecteur indépendant.

Une composition selon l'invention peut être préparée selon les procédés connus dans le domaine des vaccins et les doses applicables peuvent varier dans une large gamme. Elles sont fonctions notamment des polypeptides et du vecteur employés, de la pathologie à traiter, de l'état du patient et d'autres paramètres qui peuvent être évalués par le clinicien. Cependant, en général, la dose de virus sera de 10⁴ à 10¹³, avantageusement de 10⁵ à 10¹² et, de préférence de 10⁵ à 10⁹ unités formant des plages (ufp) lorsque l'agent thérapeutique est un vecteur viral et de 0,05 à 500 mg, avantageusement de 0,5 à 200 mg et, de préférence de 1 à 100 mg lorsque l'agent thérapeutique est d'origine polypeptidique.

Une composition selon l'invention peut être administrée selon n'importe quelle voie conventionnelle d'administration, de préférence systémique et en particulier par voie intramusculaire, intraveineuse, intrapulmonaire, sous-cutanée ou sous-épithéliale ou bien par scarification. Dans le cas d'une tumeur accessible, il est également possible de recourir à une injection directe dans le site ou à proximité de la tumeur ou à une application topicale. A titre de vaccin, une composition selon l'invention peut être administrée selon les pratiques courantes dans le domaine, par exemple en dose unique ou répétée une ou plusieurs fois après un certain délai d'intervalle. Par contre dans le cadre d'un traitement curatif, elle peut être administrée fréquemment pendant une période suffisante pour que le traitement soit efficace. Lorsque l'agent thérapeutique est un vecteur viral, le virus est de préférence sous forme vivante. S'agissant d'un vecteur poxviral, on préférera employer une souche atténuée comme la souche MVA ou la souche Copenhague thymidine kinase négative. Enfin un vecteur viral recombinant peut être atténué par un traitement chimique approprié connu de l'homme du métier. Toutefois, on peut aussi envisager d'injecter un vecteur recombinant tué.

Selon un mode de mise en oeuvre préféré, une composition antitumorale selon l'invention comprend une quantité thérapeutiquement efficace de l'agent thérapeutique en association avec un support acceptable d'un point de vue pharmaceutique. Le support est choisi de manière à permettre son administration par injection à l'homme ou à l'animal. Elle peut également comprendre un véhicule, un diluant et/ou un adjuvant et se présenter sous forme liquide ou lyophilisée. A cet égard, l'association à une ou plusieurs substances susceptibles d'améliorer l'efficacité transfectionnelle et/ou la stabilité du vecteur peut être envisagée. Ces substances sont largement documentées dans la littérature accessible à l'homme de l'art (voir par exemple Felgner et al., 1989, Proc. West. Pharmacol. Soc. *32*, 115-121 ; Hodgson et Solaiman, 1996, Nature Biotechnology *14*, 339-342 ; Remy et al., 1994, Bioconjugate chemistry *5*, 647-654). A titre illustratif et non limitatif, il peut s'agir de polymères, de lipides notamment cationiques, de liposomes, de protéines nucléaires et de lipides neutres. Une combinaison envisageable est un vecteur plasmidique associé à des lipides cationiques (DC-Chol, DOGS ... etc) et des lipides neutres (DOPE). La composition peut également être associée à d'autres substances, notamment anti-cancéreuses, celles-ci pouvant être administrées séparément ou de façon concomitante. Le ligand Flt3 est un exemple parmi d'autres (Lynch et al., 1997, Nature Medicine *3*, 625 ; Brasel et al., 1996, Blood *88*, 2004-2012 ; Marakovsky et al., 1996, J. Exp. Med. *184*, 1953-1962). On indique que la composition peut être sous forme de pseudoparticules lorsqu'elle comprend les polypeptides L1 et/ou L2.

La présente invention a également pour objet un vecteur recombinant comprenant au moins les séquences codant pour un polypeptide immunogène originaire d'une région précoce et/ou tardive d'un papillomavirus, ledit polypeptide ayant les caractéristiques définies ci-avant. Il peut en outre porter d'autres séquences d'intérêt, par exemple codant pour un ou plusieurs autres peptides immunogènes et/ou immunostimulateurs, tels que décrits auparavant.

Le choix d'un vecteur selon l'invention est large. Il peut s'agir d'un vecteur plasmidique ou viral tel que ceux cités ci-dessus. Un mode de réalisation préféré consiste en un vecteur poxviral et tout particulièrement un virus de la vaccine de la souche Copenhague ou MVA. Les sites d'insertion et les éléments nécessaires à l'expression des séquences d'intérêt à exprimer peuvent être choisis parmi ceux mentionnés précédemment.

Un vecteur préféré est un virus de la vaccine de la souche Copenhague ou MVA dans lesquels sont insérées :
(1) les séquences codant pour un polypeptide immunogène ayant une séquence homologue ou identique à celle montrée dans la SEQ ID NO: 1,
(2) les séquences codant pour un polypeptide immunogène ayant une séquence homologue ou identique à celle montrée dans la SEQ ID NO: 2,
(3) les séquences codant pour un polypeptide immunogène ayant une séquence homologue ou identique à celle montrée dans la SEQ ID NO: 1 et pour un polypeptide immunogène ayant une séquence homologue ou identique à celle montrée dans la SEQ ID NO: 2,
(4) les séquences codant pour un polypeptide immunogène ayant une séquence homologue ou identique à celle montrée dans la SEQ ID NO: 1, pour un polypeptide immunogène étant la protéine L1 d'un papillomavirus et/ou pour un polypeptide immunogène étant la protéine L2 d'un papillomavirus,
(5) les séquences codant pour un polypeptide immunogène ayant une séquence homologue ou identique à celle montrée dans la SEQ ID NO: 2, pour un polypeptide immunogène étant la protéine L1 d'un papillomavirus et/ou pour un polypeptide immunogène étant la protéine L2 d'un papillomavirus, ou
(6) les séquences codant pour un polypeptide immunogène ayant une séquence homologue ou identique à celle montrée dans la SEQ ID NO: 1, pour un polypeptide immunogène ayant une séquence homologue ou identique à celle montrée dans la SEQ ID NO: 2, pour un polypeptide immunogène étant la protéine L1 d'un papillomavirus et/ou pour un polypeptide immunogène étant la protéine L2 d'un papillomavirus.

Il peut de manière optionnelle également inclure les séquences codant pour l'IL2 ou le polypeptide B7.1

La présente invention concerne également une particule virale préparée à partir d'un vecteur viral recombinant selon l'invention. S'agissant d'un vecteur adénoviral, les particules virales peuvent être générées par transfection du vecteur dans une cellule de complémentation adaptée à ses déficiences. On aura par exemple recours à la lignée 293 établie à partir de cellules de rein embryonnaire humain, qui complémente efficacement la fonction E1 (Graham et al., 1977, J. Gen. Virol. *36*, 59-72), la lignée A549-E1 (Imler et al., 1996, Gene Therapy *3*, 75-84) ou une lignée permettant une double complémentation (Yeh et al., 1996, J. Virol. *70*, 559-565 ; Krougliak et Graham, 1995, Human Gene Therapy *6*, 1575-1586 ; Wang et aL, 1995 Gene Therapy *2*, 775-783 ; demande internationale WO 97/04119). Par cellule de complémentation, on entend une cellule capable de complémeuter *en trans* un vecteur défectif pour générer une particule virale. Ladite cellule peut ne pas complémenter à elle seule toutes les fonctions défectives du vecteur et dans ce cas on peut avoir recours à un virus auxilliaire pour une complémentation partielle. La particule virale peut être récupérée du surnageant de culture mais également des cellules. Une des méthodes couramment employée consiste à lyser les cellules par des cycles consécutifs de congélation/décongélation pour recueillir les virions dans le surnageant de lyse. Ceux-ci peuvent être amplifiés et purifiés selon les techniques de l'art (procédé chromatographique, ultracentrifugation notamment à travers un gradient de chlorure de césium ...).

Une particule rétrovirale peut être obtenue par transfection du vecteur rétroviral dans une lignée appropriée, par exemple une lignée capable de fournir *en trans* les polypeptides viraux gag, pol et/ou env dont les séquences sont délétées /non fonctionnelles dans le vecteur. De telles lignées sont décrites dans la littérature (PA317, Psi CRIP GP + Am-12 etc...)

S'agissant d'un vecteur poxviral, les conditions générales d'obtention d'un virus de la vaccine capable d'exprimer un gène hétérologue sont enseignées dans le brevet européen EP 83 286 et la demande EP 206 920. Quant au virus MVA, il est plus particulièrement décrit dans Mayr et al. (1975, Infection 3, 6-14) et Sutter et Moss (1992, Proc. Natl. Acad. Sci. USA *89*, 10847-10851). Brièvement, le(s) gène(s) à transférer placé(s) sous le contrôle des éléments appropriés pour son (leur) expression *in vivo* est (sont) inséré(s) dans un vecteur de transfert incluant des séquences virales de part et d'autre du site d'insertion. Il est introduit dans des cellules infectées par un virus de la vaccine infectieux. Le gène recombinant est intégré dans le génome viral par recombinaison homologue entre les séquences homologues du virus infectieux et du vecteur de transfert.

Le vecteur recombinant ou la particule virale peut être éventuellement associé à une ou plusieurs substances améliorant l'efficacité transfectionnelle et/ou la stabilité du vecteur déjà citées.

Dans le cadre de la présente invention, ledit polypeptide immunogène peut être ancré dans la structure protéique entourant la particule virale (capside, enveloppe...).

Les poxvirus sont des structures complexes entourées par de multiples membranes. Deux types de particules virales sont produites : les virions matures intracellulaires (IMV) et les virions enveloppés extracellulaires (EEV). La surface des IMV est composée de 2 membranes superposées et l'enveloppe des EEV consiste en 4 membranes incluant la membrane plasmique. Conformément aux buts poursuivis par la présente invention, les séquences codant, pour le polypeptide immunogène peuvent également être modifiées en vue d'une insertion dans l'une ou l'autre des membranes poxvirales (IMV ou EEV) dans le but d'améliorer la réponse immune. Selon un mode de réalisation avantageux, le peptide immunogène est ancré dans la membrane externe de l'enveloppe d'une particule EEV (Katz et al. 1997, AIDS Res. Hum. Retrov. *13*, 1497-1500). Pour ce faire, les séquences codant pour la partie C-terminale de la protéine B5R, constituant protéique de ladite membrane externe, sont insérées en 3' de la région codante du polypeptide immunogène juste en amont du codon STOP. Un exemple tout à fait préféré est une fusion entre le polypeptide E7 ou un de ses mutants non oncogènes et les 42 résidus C-terminaux de la protéine B5R. L'effet bénéfique sur la réponse immune peut être évalué par des études d'immunoprophylaxie et d'immunothérapie selon le protocole décrit dans les exemples qui suivent comparant les différentes formulations (localisation native, ancrage dans la membrane cellulaire, ancrage dans la membrane virale).

La présente invention concerne également une composition antitumorale caractésisée en ce qu'elle comporte un ou plusieurs polypeptides immunogènes, l'un au moins desdits polypeptides présentant les caractéristiques définies ci-avant pour les compositions antitumorales selon la présente invention comprenant au moins un vecteur recombinant.

La présente invention concerne également l'utilisation d'une composition antitumorale, d'un vecteur recombinant ou d'une particule virale selon l'invention, pour la préparation d'un médicament pour le traitement ou la prévention du cancer ou de tumeurs et, en particulier du cancer du col de l'utérus, d'une dysplasie du col de bas grade ou d'une infection à papillomavirus. L'utilisation préférée est pour la préparation d'un médicament injectable par la voie intramusculaire.

Enfin, on peut citer une méthode de traitement ou de prévention des pathologies citées ci-dessus, selon laquelle on administre à un individu ayant besoin d'un tel traitement une quantité efficace d'un point de vue pharmaceutique d'une composition antitumorale, d'un vecteur recombinant ou d'une particule virale selon l'invention.

La présente invention est illustrée par référence aux figures suivantes.

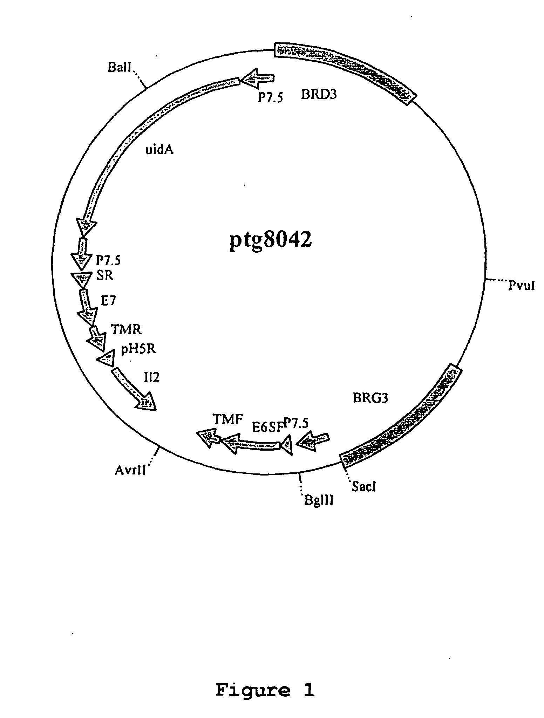

La Figure 1 est une représentation schématique de pTG8042. BRG3 et BRD3 représentent les bras de recombinaison gauche et droit permettant l'insertion au niveau de la zone d'excision III du virus MVA. pTG8042 comprend une cassette d'expression du gène E6 de HPV16 fusionné à la séquence signal (SF) et la région transmembranaire (TMF) de la protéine F du virus de la rougeolé, dirigé par le promoteur 7.5k ; une cassette d'expression du gène IL-2 placé sous le contrôle du promoteur pH5R ; une cassette d'expression du gène E7 de HPV16 fusionné à la séquence signal (SR) et la région transmembranaire (TMR) de la glycoprotéine rabique, placé sous le contrôle du promoteur p7.5 et une cassette d'expression du gène *gusA* (uidA) placé sous le contrôle du promoteur p7.5

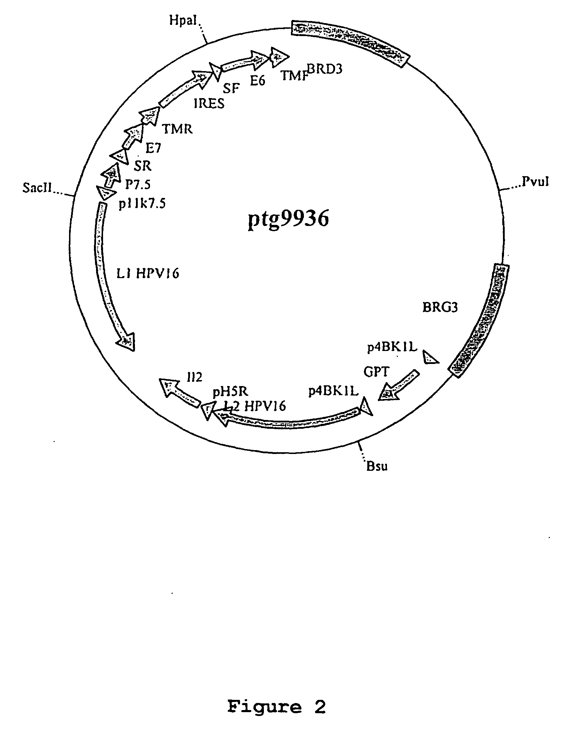

La Figure 2 est une représentation schématique du vecteur pTG9936. BRG3 et BRD3 représentent les bras de recombinaison gauche et droit permettant l'insertion au niveau de la zone d'excision III du génome MVA Le pTG9936 porte le promoteur p4BKIL dirigeant l'expression du gène marqueur *gpt* le promoteur p4BKIL dirigeant l'expression du gène L2 de HPV16, le promoteur pH5R dirigeant l'expression de l'IL-2, le promoteur p11k7.5 dirigeant l'expression du gène L1 de HPV16, le promoteur p7.5 dirigeant l'expression du gène E7 de HPV16 fusionné à la séquence signal (SR) et la région transmembranaire (TMR) de la glycoprotéine du virus rabique suivi d'une séquence IRES et du gène E6 de HPV16 fusionné à la séquence signal (SF) et la région transmembranaire (TMF) de la protéine F du virus de la rougeole.
La Figure 3 illustre l'expérience N121 en présentant le pourcentage de survie en fonction du temps (en jours) des animaux vaccinés avant épreuve tumorale avec le virus MVATG8042, MVATG6090, MVAN33 ou avec du PBS.
La Figure 4 représente le pourcentage d'animaux sans tumeurs en fonction du temps (en jours) après administration de 10⁷, 10⁶ ou 10⁵ pfu de virus MVAN33 ou MVATG8042 (expérience N127).
La Figure 5 illustre l'expérience N134 en présentant le pourcentage de survie en fonction du temps (en jours) des animaux traités par le virus MVATG8042 ou par le contrôle MVAN33 par scarification, voie sous-cutanée (SC), intrapéritonéale (IP) et intramusculaire (IM).

### EXEMPLES

La présente invention est plus complètement décrite, sans pour autant être limitée, à l'aide des exemples suivants.

Les constructions décrites ci-dessous sont réalisées selon les techniques générales de génie génétique et de clonage moléculaire, détaillées dans Maniatis et al., (1989, *supra*) ou selon les recommandations du fabricant lorsqu'on utilise un kit commercial. La mutagénèse dirigée *in vitro* par oligonucléotides synthétiques est effectuée à l'aide du kit distribué par Amersham. Les techniques d'amplification par PCR sont connues de l'homme de l'art (voir par exemple PCR Protocols-A guide to methods and applications, 1990, edité par Innis, Gelfand, Sninsky et White, Academic Press Inc). S'agissant de la réparation des sites de restriction, la technique employée consiste en un remplissage des extrémités 5' protubérantes à l'aide du grand fragment de l'ADN polymérase I d'*E*. *coli* (Klenow). En ce qui concerne les étapes de clonage, les bactériophages M13 recombinants sont multipliés sur la souche *E*. *coli* NM522 (Stratagène) dans un milieu minimum gélosé (agar 7,5 %) ou dans un milieu riche LBM liquide. Les plasmides recombinants portant le gène de résistance à l'ampicilline sont repliqués dans les souches *E*. *coli* C600 (Stratagène), BJ 5183 (Hanahan, 1983, J. Mol. Biol. *166*, 557-580) et NM522 sur milieu gélosé ou liquide supplémenté de 100 µg/ml d'antibiotique. On utilise préférentiellement la souche BJ5183 lorsque le clonage est effectué par recombinaison homologue (Bubeck et al., 1993, Nucleic Acid Res. *21*, 3601-3602).

La construction des virus de la vaccine recombinants est effectuée selon la technologie classique dans le domaine divulguée dans les documents déjà cités et dans Mackett et al., (1982, Proc. Natl. Acad. Sci. USA *79*, 7415-7419) et Mackett et al. (1984, J. Virol. *49*, 857-864).

Les tests *in vivo* (immunoprophylaxie ou immunothérapie) sont réalisés sur des souris femelles C57B16 (C. Rivers Rouen, France) ou des souris nude (Janvier, Le Genest St. Isle, France) âgées de 6 à 8 semaines. Les animaux sont généralement répartis en groupes de 20 (sauf lorsqu'indiqué) selon les virus, la dose ou la voie d'administration à tester. Les cellules tumorales TC1 (Wu, John Hopkins University, Baltimore, USA) sont obtenues de cellules de poumon prélevées de souris C57 B16 transduites avec deux rétrovirus, l'un exprimant les gènes E6 et E7 natifs de HPV-16 et l'autre exprimant l'oncogène ras. Les cellules sont cultivées en milieu DMEM (Dubelcco Modified Eagles Medium) en présence de G418 (0,5 mg/ml). Les cellules sont utilisées pour l'épreuve animale après traitement à la trypsine et 3 lavages en milieu isotonique.

### EXEMPLE 1 : Construction des vecteurs portant les séquences codant pour les mutants non oncogènes de E6 et E7 de IHPV-16 munis de signaux de localisation transmembranaire.

Les gènes E6 et E7 sont isolés à partir de la lignée cellulaire Caski comme décrit aux exemples 2 et 3 du brevet européen EP 0 462 187. Deux constructions ont été dérivées du clone M13E7/E6 contenant les gènes E6 et E7 du HPV-16 afin de faciliter les étapes ultérieures de clonage. La première désignée M13TG8188 résulte de l'introduction par mutagénèse dirigée de sites *Pst*I et *Bam*HI respectivement en amont et en aval du gène E7 et la seconde, M13TG8189 comporte un site *Pst*I en amont du gène E6. L'introduction de mutations ponctuelles en amont d'un ATG initiateur et en aval d'un codon stop sont à la portée de l'homme de l'art.

L'association de la protéine E7 de HPV-16 avec le produit du gène de rétinoblastome a été démontrée par divers auteurs (voir par exemple Munger et al., 1989, EMBO J. 8, 4099-4105) et corrélée à son pouvoir transformant. Pour des raisons évidentes de sécurité, on génère un mutant non oncogène délété des codons 21 à 26 de la protéine E7 native impliqués dans la fonction de transformation, par mutagénèse dirigée du vecteur M13TG8188 à l'aide de l'oligonucléotide oTG5118 (SEQ ID NO: 3). On obtient M13TG9104 portant le gène E7 muté, désigné ci-après E7*.

Les signaux de sécrétion et d'ancrage de la glycoprotéine rabique sont isolés du gène de la glycoprotéine rabique inséré sous forme d'un fragment *Bgl*II dans le pBR327 (pTG150 décrit dans le brevet français 83 15716) et sous cloné en vecteur M13 (M13TG177). On introduit entre ces signaux un site *Bam*HI en phase avec ces derniers par mutanénèse dirigée à l'aide de l'oligonucléotide oTG5745 (SEQ ID NO: 4). La construction résultante est dénommée M13TG9128. Les séquences de sécrétion et d'ancrage sont ensuite isolées du M13TG9128 par digestion *Pst*I et insérées en 3' du promoteur naturel du virus de la vaccine p7.5 dans le vecteur pTG5003 linéarisé par *Pst*I, pour donner pTG5016. A titre indicatif, pTG5003 dérive du pTG186poly (décrit dans le brevet français 2 583 429) par digestion *Sal*I, traitement par le fragment Klenow, puis digestion par *Sma*I et religation, de sorte qu'il ne contient plus dans son polylinker que les sites *Pst*I et *Eco*RI à l'exclusion de tout autre. Le vecteur M13TG9104 est modifié par mutagénèse dirigée à l'aide des oligonucléotides oTG6390 et oTG6880 (SEQ ID NO: 5 et 6) afin de mettre en phase les séquences E7* et les signaux de sécrétion et d'ancrage de la glycoprotéine rabique (désignées par la suite E7*TMR). La construction résultante est dénommée M13TG9150.

De même, il a été démontré que la protéine E6 de HPV-16 pouvait interagir avec le produit d'expression du gène suppresseur de tumeur *p53* (Crook et al., 1991, Cell *67*, 547-556). Le domaine impliqué dans cette interaction a clairement été défini et se situe entre les résidus 111 à 115 de la protéine native. Le vecteur M13TG9125 est généré par mutagénèse de M13TG8189 à l'aide de l'oligonucléotide oTG5377 (SEQ ID NO: 7). Le gène E6 λ111-115 est désigné ci-après E6*.

Les signaux de sécrétion et d'ancrage de la protéine F du virus. de la rougeole sont isolés par PCR à partir de la construction plasmidique pTG2148 (décrite dans le brevet européen EP 0 305 229) contenant l'ADN codant pour la protéine F du virus de la rougeole. La fusion entre ces séquences et celles codant pour E6* est réalisée par PCR direct : la séquence de sécrétion est amplifiée à l'aide des oligonucléotides oTG10829 portant en 5' un site *Xba*I (SEQ ID NO: 8) et oTG10830 recouvrant l'extrémité 5' de E6 (SEQ ID NO: 9), les séquences codant pour le mutant E6* sont amplifiées à partir du M13TG9125 à l'aide des oligonucléotides oTG10835 permettant la fusion avec l'extrémité 3' du signal de sécrétion de la protéine F (SEQ ID NO: 10) et oTG10836 permettant la fusion avec l'extrémité 5' de la séquence d'ancrage de la protéine F (SEQ ID NO: 11). Pour la séquence d'ancrage, on met en oeuvre les amorces oTG10833 permettant la fusion entre le 3' de E6* et le 5' de la séquence d'ancrage (SEQ ID NO: 12) et oTG10834 crééant en 3' les sites *Kpn*I et *Sph*I (SEQ ID NO: 13). Le fragment amplifié portant les séquences E6* fusionnées en N et C terminal respectivement aux séquences de sécrétion et d'ancrage membranaire de la protéine F (désignées par la suite E6*TMF) est digéré par *Xba*I et *Sph*I puis inséré aux mêmes sites dans le vecteur M13TG131 (Kieny et al., 1983, Gene 26, 91-99). La construction ainsi obtenue est appelée M13TG9199.

### EXEMPLE 2 : Construction du virus recombinant MVATG8042 exprimant les antigènes E6 et E7 de localisation transmembranaire et l'IL-2 humaine.

Le virus MVA dérive de la souche du virus de la vaccine Ankara. Il n'est pas capable de générer des particules infectieuses sur les cellules de mammifères mais se développe correctement sur des fibroblastes embryonnaires de poulet. Son adaptation à ces cellules a provoqué l'excision de 6 régions non essentielles pour son développement et son cycle infectieux sur ce type de cellules (disparition d'environ 15 % du génome viral ; Meyer et al., 1991, J. Gen. Virol. *72*, 1031-1038). L'intégration de matériel génétique exogène peut être réalisée au niveau de l'une quelconque de ces zones d'excision. Dans le cadre de la présente invention, on utilise les excisions II et III localisées au niveau des fragments de restriction *Hin*dIII N et A respectivement (Altenburger et al., 1989, Arch. Virol. *105*, 15-27).

Dans un premier temps, on construit le vecteur pTG6019 permettant l'insertion dans la zone d'excision III du virus MVA. Les bras de recombinaison homologue de part et d'autre de la zone d'excision III sont isolés par PCR à partir du génome viral (voir brevet américain US 5,185,146) et des amorces oTG7637 et oTG7638 (SEQ ID NO: 14 et 15) pour le bras gauche et oTG7635 et oTG7636 (SEQ ID NO: 16 et 17) pour le bras droit. Les fragments amplifiés sont clonés au site *Eco*RI du vecteur pTG1E, pour donner pTG6019. Le matériel génétique à transférer est inséré entre les deux bras de recombinaison. Le vecteur pTG1E est similaire au pTG1H (brevet français 2 583 429) mis à part la présence d'un adaptateur *Eco*RI à la place de sites multiples de clonage.

On insère en premier lieu une cassette d'expression du gène marqueur *gus A*. Le promoteur 7,5K est tout d'abord cloné dans le site *Bam*HI de pTG6019. On obtient pTG6021 dans le site *Bam*HI duquel on insère le gène *gus A* généré sous forme d'un fragment *Bgl*II-*Bam*HI. Celui-ci peut être obtenu à partir de la séquence divulguée dans la littérature. La construction résultante est dénommée pTG6022. La présence du marqueur va permettre de discriminer les virus sauvages des virus recombinants par détection de l'activité enzymatique GUS par le substrat Xg1cA. Une coloration rouge révèle l'activité β-glucuronidase. Cependant, dans l'optique d'une application clinique, il peut être utile d'être en mesure d'éliminer ce marqueur bactérien du produit final après la sélection des virus recombinants. Pour ce faire, on met à profit la capacité de la vaccine à déléter les séquences comprises entre deux sites homologues. C'est pourquoi on insère un second promoteur p7,5K en aval du gène *gus A* dans une orientation sens par rapport à celui qui dirige l'expression de ce dernier. Le vecteur pTG6025 résulte de l'insertion entre les sites *Bam*HI et *Sac*I de pTG6022 d'un fragment p7,5K muni d'extrémités cohésives.

Par ailleurs, l'ADNc codant pour l'interleukine-2 humaine est isolé du plasmide pTG36 (brevet français 2 583 770) par digestion *Pst*I et inséré dans le site *Pst*I du plasmide pTG186 (brevet français 2 583 429), donnant lieu à pTG188. Le virus obtenu par recombinaison homologue est dénommé WTG188. Après digestion *Bgl*II/*Eco*RI, les séquences IL-2 sont insérées en 3' du promoteur naturel de la vaccine pH5R aux sites *Bam*HI/*Eco*RI du M13TG9132, pour donner M13TG9185. A titre indicatif, le vecteur M13TG9132 provient de l'insertion du promoteur du gène H5R isolé par PCR du génome viral dans le phage M13TG6131, lequel dérive de M13TG131 (Kieny et al., 1983, *supra*) par mutation du site *Bgl*II interne situé en dehors des sites multiples de clonage.

Les gènes HPV-16 et IL-2 sont ensuite clones dans la zone d'excision III du génome du MVA. Les séquences E7*TMR sont isolées par digestion *Bgl*II/*Hin*dIII et insérées entre les sites *Bam*HI et *Hin*dIII du pTG6025 en 3' du promoteur vaccine p7.5. La construction résultante est dénommée pTG6050. Un site d'insertion de la cassette d'expression du gène de l'IL-2 humaine par recombinaison homologue est créé entre les sites *Hin*dIII et *Kpn*I du pTG6050 par insertion des oligonucléotides oTG10502 et oTG10503 (SEQ ID NO: 18 et 19). Le vecteur généré pTG6074 est ensuite linéarisé par digestion *Hin*dIII/*Kpn*I et la recombinaison homologue avec la cassette d'expression pH5R-IL-2 isolée du M13TG9185 par digestion *Bgl*II/*Eco*RI est réalisée. La construction résultante pTG6088 est finalement linéarisée par *Kpn*I et mis en ligation avec le gène E6*TMF isolé du M13TG9199 par digestion *Kpn*I/*Xba*I et le promoteur p7.5 isolé du M13TG9136 par digestion *Xba*I/*Kpn*I. La construction résultante est dénommée pTG8042 (Figure 1). Le vecteur M13TG9136 provient de M13TG5107 modifié par mutagénèse dirigée à l'aide des oTG5925 (création d'un site *Pst*I en 3' du promoteur p7.5 ; SEQ ID NO: 20) et oTG5924 (création d'un site *Bam*HI et *Kpn*I en 5' du promoteur p7.5 ; SEQ ID NO: 21).

Le virus MVATG8042 est généré par recombinaison homologue avec le génome MVA selon les règles de l'art. L'isolement des recombinants est facilité par la présence du gène marqueur *gusA*.

On vérifie que la modification de localisation cellulaire des antigènes précoces de HPV ne nuit pas à leur expression. L'analyse par Western blot d'extraits cellulaires infectés par MVATG8042 à l'aide d'un anticorps anti-E7 permet la détection de 3 bandes d'un poids moléculaire compris entre 20 et 35 kDa. Cette hétérogénéité peut être expliquée par la présence d'un site potentiel de O-glycolysation dans la zone d'ancrage membranaire de la glycoprotéine rabique. Lorsque l'analyse Western blot est répétée en présence de Phényl-Gal-Nac (Phényl N-acétyl α D galactopyranoside, Sigma, P4023), on ne détecte qu'une seule forme de 20 kDa ce qui confirme la O-glycosylation du produit d'expression du gène E7*TMR.

La détection par Western blot à l'aide d'un anticorps anti E6 du produit d'expression du gène E6*TMF ne met en évidence qu'une seule bande migrant au poids moléculaire attendu de 20 kDa, ce qui confirme l'absence de modifications post-traductionnelles. A titre indicatif, les anticorps anti-E6 et E7 précités sont des antisérum de lapins obtenus par administration de l'antigène purifié. Mais tout autre anticorps spécifique, qu'il soit monoclonal ou polyclonal, peut également convenir.

L'expression du gène hIL-2 est évaluée par ELISA (Quantikine R&D Systems) et test de prolifération cellulaire IL-2 dépendant. Selon les conditions de culture, la quantité d'IL-2 produite varie de 200 à 800 ng/ml/24 h par 10⁶ cellules infectées avec 0,1 pfu/cellule.

### EXEMPLE 3 : Efficacité in vivo du virus MVATG8042 (immunothérapie).

Des souris C57BL6 sont inoculées avec 10³ cellules BMK-16 myc implantées en voie sous-cutanée. A titre indicatif, la lignée cellulaire dérive de cellules de rein de souris nouveau-nées transfectées par le génome HPV-16 et le gène c myc murin. 10⁷ pfu (unités formant des plages) de virus MVATG8042 sont ensuite administrés également en sous-cutanée à J3, J6 et J9 et l'évolution des tumeurs suivie régulièrement. Les souris traitées présentent un retard de la croissance tumorale jusqu'à J15 par rapport aux contrôles constitués par des animaux ayant reçu un virus MVA non recombinant. De plus, le traitement s'accompagne d'une régression partielle des tumeurs à J30-35 qui n'est pas observée lorsqu'on met en oeuvre un MVA équivalent exprimant les mutants E6* et E7* de localisation nucléaire native.

### EXEMPLE 4 : Construction du virus recombinant VVTG5095 exprimant l'antigène E7* de localisation transmembranaire.

Les séquences E7*TMR sont isolées du M13TG9150 par digestion *Bgl*II/*Bam*HI et insérées au site *Bam*HI du pTG5016. La construction résultante, dénommée pTG5095, contient les séquences E7* fusionnées aux signaux de sécrétion et d'ancrage de la glycoprotéine rabique, sous contrôle du promoteur p7.5. Le virus VVTG5095 est généré par recombinaison homologue avec le génome vaccine.

### EXEMPLE 5 : Construction du virus recombinant VVTG6002 exprimant l'antigène E7* de localisation transmembranaire et l'immunostimulateur B7.1.

Le gène B7.1 humain est isolé à partir d'une lignée cellulaire humaine Daudi par PCR réverse (RT-PCR) à l'aide des amorces oTG6353 et oTG6352 (SEQ ID NO: 22 et 23) et inséré entre les sites *BglI*I et *Eco*RI de M13TG6131. On obtient M13TG9149 dont on isole le fragment *Bgl*II*-Eco*RI lequel est sous cloné entre les mêmes sites de M13TG5107, en 3' du promoteur p7.5 (M13TG5107 porte les séquences promotrices p7.5 clonées dans le site *Bam*HI du M13TG130). La cassette p7.5-B7.1 est isolée de la construction ainsi obtenue dénommée M13TG9152 par digestion *Eco*RI/*Pst*I et introduite dans le site *Eco*RI de pTG5095 à l'aide de l'oTG1086 (5'AATTTGCA3'). La construction résultante est dénommée pTG6002 et les virus recombinants VVTG6002 sont produits par recombinaison homologue avec le génome de la vaccine.

Une construction équivalente est réalisée par insertion des cassettes d'expression des gènes E7* et B7.1 dans la zone d'excision III du génome MVAN33 selon la même technologie que celle développée à l'exemple 2.

### EXEMPLE 6 : Efficacité in vivo des virus VVTG5095 et VVTG6002 (immunoprophylaxie).

Des souris C57BL6 ont été vaccinées à trois reprises par voie sous-cutanée avec 10⁷ pfu de VVTG5095 ou VVTG6002. Trois jours après la dernière immunisation, ces animaux sont éprouvés avec 10³ cellules E7W1 implantées en sous-cutané. A titre indicatif, les cellules E7W1 proviennent d'une lignée de lymphome murin transfectée par un vecteur exprimant le gène E7 oncogène de HPV-16. Le pourcentage de survie des animaux en fonction du temps est comparé à celui obtenu avec des souris témoins traitées avec 10⁷ pfu d'un virus de la vaccine non recombinant VVTG186 (dérivé du vecteur TG186 décrit ci-dessus). Le suivi de la mortalité montre une différence entre les trois groupes. Alors que dans le groupe témoin 100 % des animaux sont morts à J36, on observe la survie d'environ un quart des animaux vaccinés par VVTG5095. La protection est sensiblement améliorée avec la construction VVTG6002 qui inclut les séquences codant pour l'antigène B7.1.

### EXEMPLE 7: Construction de MVATG9936 exprimant les gènes précoces modifiés et les gènes tardifs de HPV 16.

Les fragments codant pour les protéines L1 et L2 de HPV16 sont isolés par PCR à partir d'ADN génomique de cellules Caski (ATCC 1550) selon les techniques générales de l'art. Le fragment d'amplification portant les séquences L1 est sous cloné dans M13TG130 (Kieny et al., 1983, Gene *26*, 91-99), pour donner la construction M13TG8171. La séquence du gène L1 clone révèle plusieurs mutations par rapport à la séquence contenue dans Genebank (accession K02718) : C à la place d'un A en position 248, C à la place d'un A en position 253, G à la place d'un A en position 537, G à la place d'un C en position 682, G à la place d'un A en position 874, insertion d'un triplet ACT en position 1393, délétion d'un triplet GAT en position 1390. La séquence est corrigée par mutagénèse ponctuelle de manière à la rendre conforme à celle publiée par Zhou et al. (1991, Virology *185*, 251-257) et introduire des mutations silencieuses au niveau des séquences TTTTTNT qui constituent des sites potentiels de terminaison de la transcription précoce susceptibles d'interférer avec la phase précoce de développement de la vaccine. La technique de mutagénèse dirigée est à la portée de l'homme de l'art. Le vecteur portant la séquence corrigée est désigné M13TG4041.

L'insertion du fragment PCR portant les séquences L2 dans le vecteur M13TG6131 conduit à M13TG9126. On dénombre 5 mutations ponctuelles par rapport à la séquence divulguée dans Genebank : C à la place d'un T en position 378, A à la place d'un G en position 691, A à la place d'un G en position 702, G à la place d'un A en position 990 et C à la place d'un A en position 1092. A titre indicatif, le vecteur M13TG6131 dérive de M13TG131 (Kieny et al., 1983, Gene *26*, 91-99) par mutation du site *Bgl*II interne situé en dehors des sites multiples de clonage.

Le vecteur M13TG4041 est digeré par *Xba*I-*Sac*I et le fragment portant les séquences L1 est inséré entre les sites *Xba*I-*Sac*I de M13TG9126 en aval du gène L2 et en direction opposée. La construction ainsi générée est dénommée M13TG4042. Puis, les séquences L1 et L2 sont isolées par digestion *Bgl*II et sous clonées entre les sites *Bgl*II et *Bam*HI du vecteur M13TG4052 contenant le promoteur synthetique p11K7.5, qui résulte de la fusion des promoteurs tardif p11K et précoce p7.5. Des deux orientations, on sélectionne celle qui place le gène L1 en aval du promoteur , qui est désignée M13TG4055. Puis, la cassette d'expression portant le promoteur pH5R et le gène hIL-2, est isolée de pTG8042 par digestion *Hin*dIII avant d'être introduite dans le site *Hin*dIII de M13TG4055 situé entre les gènes L1 et L2. Enfin, la construction obtenue, M13TG4057, est digérée par *Bgl*II et le fragment portant les séquences tardives de HPV16 est inséré dans le site *Bam*HI de M13TG4060, lequel résulte du clonage du promoteur synthétique p4BK1L. Ce dernier est un promoteur hybride entre le promoteur précoce p4B (Davidson et Moss, 1989, J. Mol. Biol. *210*, 749-769) et tardif pK1L (Davidson et Moss, 1989, J. Mol. Biol. *210*, 771-784). On obtient M13TG4062 qui comprend le gène L1 sous le contrôle de p11K7.5, la cassette pH5R-IL2 et le gène L2 sous le contrôle de p4BK1L en orientation réverse par rapport aux séquences L1.

On construit une cassette polycistronique contenant les séquences codant pour E7*TMR et pour E6*TMF. En premier lieu, les séquences IRES du virus EMC (encephalomyocardiovirus ; Genbank accession M22458) sont isolées par les techniques conventionnelles sous forme d'un fragment *Eco*RI*-Nco*I introduit en aval du gène E7*TMR entre les sites *Eco*RI et *Nco*I de pTG6002 (exemple 5), pour donner pTG8084. Puis le gène E6*TMF est amplifié par PCR à l'aide d'amorces appropriées crééant un site *Nco*I à son extrémité 5'. Le fragment *Bgl*II-*Nco*I portant le gène E7*TMR et les séquences IRES obtenu de pTG8084 et le fragment PCR portant le gène E6*TMF digéré par *Nco*I et *Sac*I sont réassemblés dans le vecteur M13TG6131 (exemple 2) préalablement digéré par *Bgl*II et *Sac*I. On obtient M13TG4059. Puis le bloc E7*TMR-IRES-E6*TMF est isolé de ce dernier sous forme d'un fragment *Bgl*II et inséré dans le site *Bam*HI de pTG8093 en aval du promoteur p7.5K. (pTG8093 résulte du clonage du promoteur p7.5K dans le vecteur pTG6025. La construction ainsi obtenue est désignée pTG9901.

Le fragment *Sac*I de M13TG4062 est cloné dans le site *Sac*I de pTG9901, pour donner pTG9902. Le gène de sélection *gpt* (Falkner et Moss, 1988, J. Virol. *62*, 1849-1854) est assemblé avec les gènes tardifs et IL2 dans le vecteur p poly II (Lathe et al., 1987, Gène *57*, 193-201) de la façon suivante. Le premier est isolé de M13TG4076 (ce vecteur contient le gène *gpt* flanqué à ses deux extrémités des séquences promotrices p4BKIL) par digestion *Bgl*II-*Nco*I et les seconds de pTG9902 par digestion *Nco*I-*Sac*I. Les fragments purifiés sont clonés entre les sites *Bgl*II-*Sac*I de p poly II. On obtient pTG9933 duquel on isole le fragment *Sac*I qui est introduit dans le vecteur pTG9901 clivé par *Sac*I. La construction ainsi obtenue pTG9936 est illustrée à la Figure 2.

Les virus MVATG9936 sont générés par recombinaison homologue avec le génome MVAN33. L'isolement de clones peut être réalisé selon les règles de l'art.

### EXEMPLE 8: Efficacité des virus en immunoprophylaxie.

### A. Importance de la formulation des virus (expérience N121)

Le but de cette étude préclinique est de comparer la souche virale (virus de la vaccine Copenhagen contre MVA) et la formulation (présentation transmembranaire contre localisation nucléaire native) en terme de protection anti-tumorale.

Des souris C57B16 sont vaccinées à trois reprises avec 10⁷ pfu de virus. Les injections sont faites tous les dix jours (J1, J11 et J21) par voie intrapéritonéale. Les animaux sont éprouvés 7 jours après la dernière immunisation par administration sous-cutanée dans leur flanc droit de 5x 10⁴ cellules TC1. Huit groupes de 20 animaux sont constitués en fonction du virus ou de la solution administré, respectivement :
1 MVATG8042 (exemple 2, E6*TMF, E7*TMR, IL-2),
2 MVATG6037 (exemple 5, E7*TMR, B7.1),
3 WTG5095 (exemple 4, E7*TMR),
4 MVATG6090 (E6*, E7*, IL-2),
5 VVTG5061x188 (E6*, E7*, IL-2),
6 VVTG186 (virus de la vaccine non recombinant),
7 MVAN33 (virus MVA non recombinant), et
8 une solution saline (PBS).

Les groupes 1 à 3 sont vaccinés avec les virus de la présente invention exprimant au moins un antigène HPV transmembranaire alors que les groupes 4 et 5 ont reçu des virus exprimant les antigènes précoces de HPV16 de localisation nucléaire native et les groupes 6 à 8 des virus contrôles non recombinants ou une solution saline. On indique que les virus MVATG6090 et VVTG5061x188 sont décrits dans la demande internationale WO98/04705. Le pourcentage de survie des animaux des différents groupes est suivi pendant les 12 semaines qui suivent l'épreuve tumorale. Les résultats peuvent être résumés de la façon suivante.

D'une manière générale, la mortalité est importante dans les trois groupes témoins atteignant 95% (avec les virus MVAN33 et W186) et 81% (avec le PBS).

On observe un accroissement significatif de la survie des animaux immunisés avec les virus exprimant les antigènes nucléaires de HPV. Ainsi, 55% des souris ayant reçu le virus MVATG6090 sont libres de tumeurs alors que l'administration de VVTG5061x188 induit un taux de réjection tumorale de 75%.

En revanche, la grande majorité des animaux vaccinés avec les virus exprimant les antigènes transmembranaires de HPV16 ont rejetés leur tumeur ou présentent un retard de croissance tumorale important. Plus précisemment, on observe 100% de rejection avec MVATG8042, 95% avec MVATG6037 et 90% avec VVTG5095.

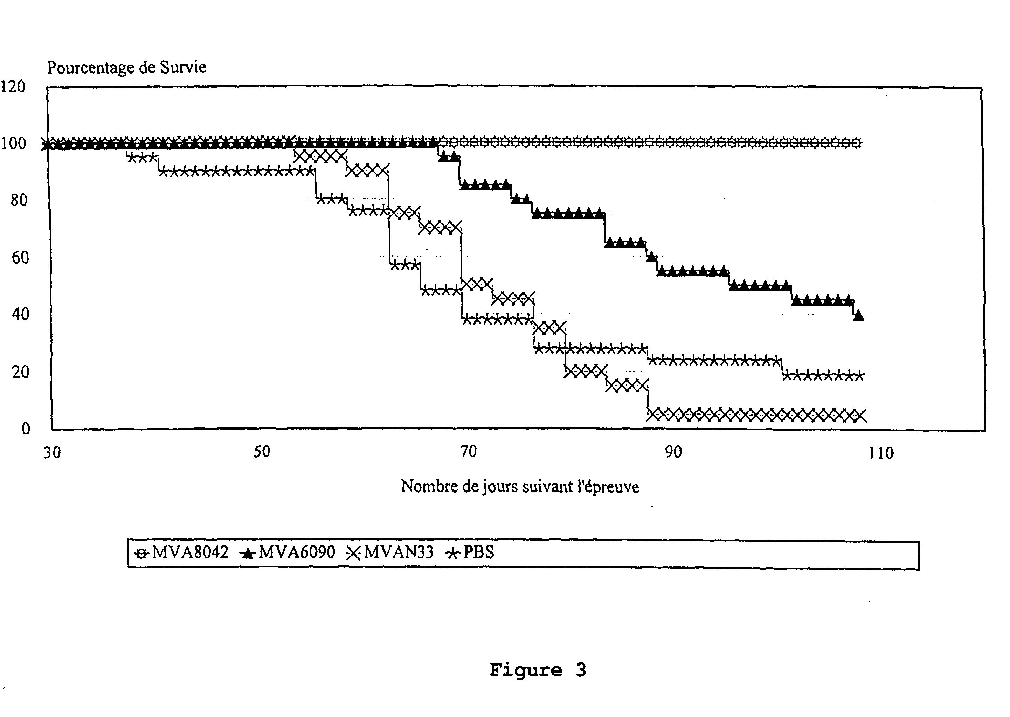

La Figure 3 présente les courbes de survie obtenues avec les animaux vaccinés avec MVATG8042 et MVATG6090 par rapport aux contrôles (MVAN33 et PBS).

Dans leur ensemble, ces données mettent en évidence l'absence de différence significative entre les virus issus d'un virus de la vaccine Copenhagen et d'un MVA et la meilleure immunogénécité conférée par la présentation membranaire. De tous les virus testés, le virus MVATG8042 est le plus efficace puisqu'il donne lieu à 100% de réjection tumorale dans ce modèle animal d'immunoprophylaxie.

### B. Etude de la réponse mémoire (expérience N122)

Les souris C57B16 sont immunisées par voie intrapéritonéale avec 10⁷ pfu de virus à J1, J11 et J21 avant d'être éprouvées à J82 par administration sous-cutanée dans leur flanc droit de 5x10⁴ cellules TC1. Huit groupes identiques à l'expérience précédente (N121) sont constitués. L'évolution des tumeurs est suivie en fonction du temps. Les données obtenues 50 jours après l'épreuve tumorale confirment que le virus le plus efficace en terme de réjection tumorale est le virus MVATG8042. Son administration protège 100% des animaux, indiquant sa capacité à induire une immunité à long terme.

### C. Effet de dose (expérience N127).

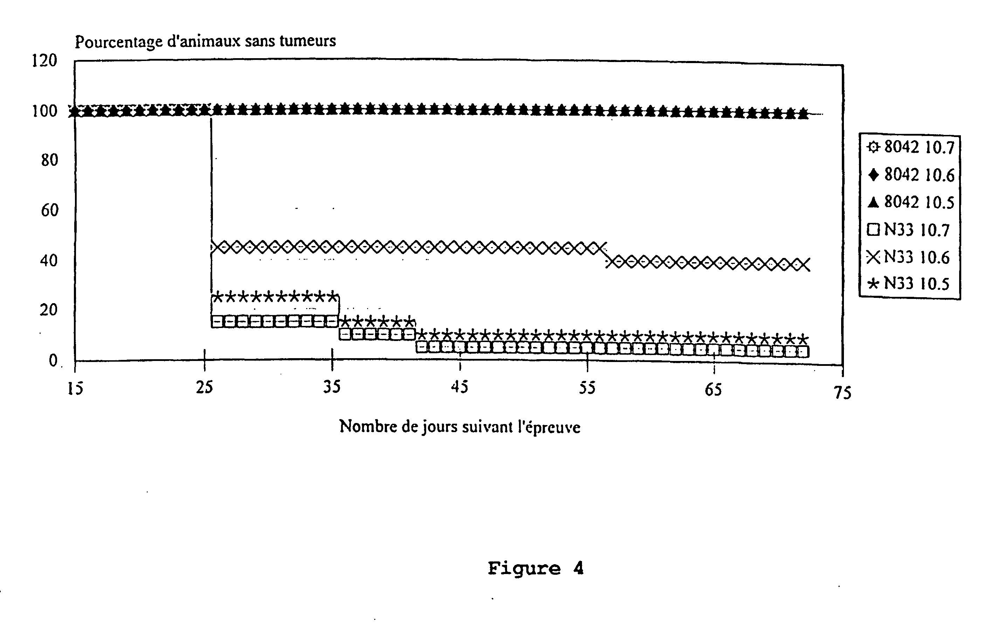

Les souris C57B16 sont immunisées par voie intrapéritonéale à J1, J11 et J21 avec 10⁵, 10⁶ ou 10⁷ pfu de virus MVATG8042 avant d'être éprouvées à J28 par administration sous-cutanée dans leur flanc droit de 5x10⁴ cellules TC1. Les animaux contrôles reçoivent des quantités identiques de MVAN33. L'évolution des tumeurs est suivie deux fois par semaine pendant 12 semaines. Les résultats (Figure 4) montrent 100% de protection quelle que soit la dose de MVATG8042 administrée alors que la grande majorité des animaux témoins développent des tumeurs. Ces données confirment l'efficacité du virus MVATG8042 même à faible dose.

### D. Effet de la voie d'administration (expérience N134).

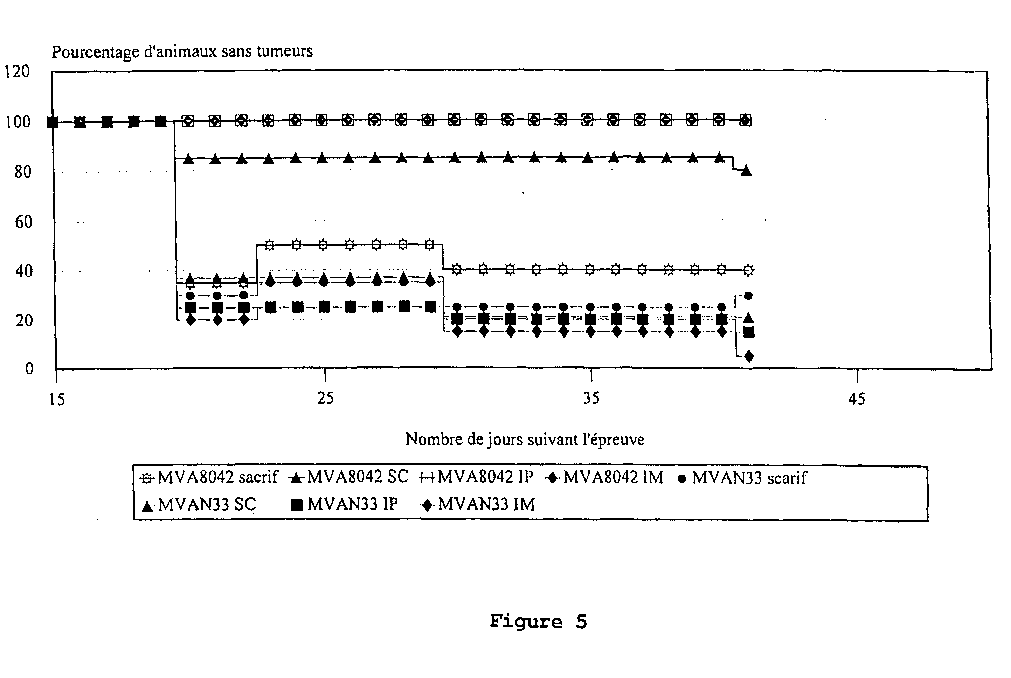

Les souris C57B16 sont immunisées par différentes routes d'administration (scarification , sous-cutanée, intrapéritonéale ou intramusculaire) à J1, J11 et J21 avec 10⁷ pfu de virus avant d'être éprouvées à J44 par administration sous-cutanée dans leur flanc droit de 5x10⁴ cellules TC1. L'évolution des tumeurs est suivie deux fois par semaine pendant 12 semaines. Comme montré à la Figure 5, les voies intrapéritonéale ou intramusculaire donnent les taux de protection les plus élevés avec le virus MVATG8042.

### EXEMPLE 9: Efficacité des virus en immunothérapie.

### A. Efficacité des virus dans un contexte d'immunothérapie (expérience N125).

Le but de cette étude est de comparer les capacités thérapeutiques à l'égard d'une tumeur préétablie des virus présentant les antigènes HPV sous une forme membranaire ou nucléaire. Pour ce faire, 5x10⁴ cellules TC1 sont administrées par voie sous-cutanée dans le flanc droit des souris C57B16 (J0). Puis, 10⁷ pfu de virus sont injectés par voie intrapéritonéale à J7, J14 et J21. Quatre groupes d'animaux sont constitués selon le virus administré : respectivement MVAN33 (contrôle négatif), une solution saline de Tris-HCl/NaCl (contrôle négatif), MVATG8042 (formulation transmembranaire) et MVATG6090 (formulation nucléaire). L'évolution des tumeurs est évaluée deux fois par semaine pendant 12 semaines. Les résultats montrent une meilleure efficacité de la présentation membranaire en terme de protection anti-tumorale dans un contexte thérapeutique. 100% des souris ayant reçu MVATG8042 ont survécu 140 jours après l'implantation des cellules tumorales alors que le pourcentage est d'environ 60% pour les animaux injectés avec MVATG6090 et bien inférieur pour les animaux contrôles.

### B. Etudes de toxicité - Effet de la dose.

Il est important de vérifier l'absence de virulence des virus avant d'envisager leur application aux tumeurs humaines. On administre à des souris nude (5 animaux/groupe) 10⁶ ou 10⁷ pfu de virus MVATG8042, MVAN33 ou un virus de la vaccine Copenhagen sauvage (VVwt) par voie intracraniale. La survie des souris est suivie pendant 20 jours.
et les résultats présentés dans le tableau 1 suivant

**Tableau 1**

| virus | Nombre de souris nude survivantes | |
|---|---|---|
| | 10⁶ pfu | 10⁷ pfu |
| MVAN33 | 5/5 | 5/5 |
| MVATG8042 | 5/5 | 5/5 |
| VVwt | - | 0/5 |

Aucun effet secondaire n'est détecté suite à l'administration intracraniale de 10⁷ pfu du virus MVATG8042 alors que toutes les souris traitées avec le virus de la vaccine sauvage sont mortes trois jours après l'injection. Le virus MVA contrôle n'est pas non plus toxique pour les animaux, ce qui confirme son atténuation par rapport au virus de la vaccine déjà décrite dans la littérature.

## Revendications

1. Composition antitumorale comprenant au moins un vecteur recombinant renfermant les séquences codant pour un ou plusieurs polypeptide(s) immunogène(s), **caractérisée en ce que** l'un au moins desdits polypeptides est un polypeptide présentant naturellement une localisation non membranaire et est modifié par insertion d'une séquence d'ancrage membranaire et, au cas où le polypeptide immunogène natif en est dépourvu, d'une séquence de sécrétion de manière à présenter une localisation membranaire à la surface des cellules dans lesquelles il est exprimé, et **caractérisée en ce que** ledit polypeptide immunogène présente un degré de similarité supérieur à 70% avec tout ou partie d'un polypeptide codé par une région précoce et/ou tardive d'un papillomavirus.

2. Composition antitumorale selon la revendication 1, **caractérisée en ce que** ledit polypeptide présente naturellement une localisation nucléaire et est en outre délété de sa séquence naturelle de localisation nucléaire.

3. Composition antitumorale selon l'une des revendications 1 et 2, **caractérisée en ce que** ladite séquence d'ancrage membranaire et/ou la séquence de sécrétion est sélectionnée parmi le groupe constitué par celle de la glycoprotéine rabique, de la glycoprotéine env du virus HIV et de la protéine F du virus de la rougeole.

4. Composition antitumorale selon l'une des revendications 2 à 3, caractirisée en ce que ladite séquence de sécrétion est insérée à l'extrémité N-terminale et la séquence d'ancrage membranaire est insérée à l'extrémité C-terminale dudit polypeptide immunogène.

5. Composition antitumorale selon l'une des revendications 1 à 4, **caractérisée en ce que** ledit polypeptide immunogène présente un degré de similarité supérieur a 70% avec polypeptide codé par la région précoce d'un papillomavirus.

6. Composition antitumorale selon la revendication 5, **caractérisée en ce que** ledit polypeptide immunogène est un polypeptide E6 ou E7 d'un papillomavirus.

7. Composition antitumorale selon la revendication 6, **caractérisée en ce que** ledit polypeptide immunogène est un variant non oncogène dudit polypeptide E6 ou E7 d'un papillomavirus, muté au niveau de la région impliquée dans le processus de transformation cellulaire.

8. Composition antitumorale selon l'une des revendications 1 à 4, **caractérisée en ce que** ledit polypeptide immunogène présente un degré de similarité supérieur à 70% avec polypeptide L1 ou L2 d'un papillomavirus.

9. Composition antitumorale selon l'une des revendications 1 à 8, **caractérisée en ce qu'**au moins un polypeptide immunogène présente un degré de similarité supérieur à 70% avec un polypeptide précoce et au moins un polypeptide immunogène présente un degré de similarité supérieur à 70% avec un polypeptide tardif d'un papillomavirus.

10. Composition antitumorale selon l'une des revendications 1 à 9, **caractérisée en ce qu'**au moins un polypeptide immunogène est tel que :
(1) ledit polypeptide immunogène a une séquence présentant un degré d'identité supérieur à 70% ou identique à celle montrée dans la SEQ ID NO: 1,
(2) ledit polypeptide immunogène a une séquence présentant un degré d'identité supérieur à 70% ou identique à celle montrée dans la SEQ ID NO: 2,
(3) ledit polypeptide immunogène a une séquence présentant un degré d'identité supérieur à 70% ou identique à celle montrée dans la SEQ ID NO: 1 et un polypeptide immunogène ayant une séquence présentant un degré d'identité supérieur à 70% ou identique à celle montrée dans la SEQ ID NO: 2,
(4) ledit polypeptide immunogène a une séquence présentant un degré d'identité supérieur à 70% ou identique à celle montrée dans la SEQ ID NO: 1, un polypeptide immunogène étant la protéine L1 d'un papillomavirus et/ou un polypeptide immunogène étant la protéine L2 d'un papillomavirus,
(5) ledit polypeptide immunogène a une séquence présentant un degré d'identité supérieur à 70% ou identique à celle montrée dans la SEQ ID NO: 2, un polypeptide immunogène étant la protéine L1 d'un papillomavirus et/ou un polypeptide immunogène étant la protéine L2 d'un papillomavirus, ou
(6) ledit polypeptide immunogène a une séquence présentant un degré d'identité supérieur à 70% ou identique à celle montrée dans la SEQ ID NO: 1, un polypeptide immunogène ayant une séquence présentant un degré d'identité supérieur à 70% ou identique à celle montrée dans la SEQ ID NO: 2, un polypeptide immunogène étant la protéine L1 d'un papillomavirus et/ou un polypeptide immunogène étant la protéine L2 d'un papillomavirus.

11. Composition antitumorale selon l'une des revendications 1 à 10, **caractérisée en ce que** ledit papillomavirus est un papillomavirus humain tel que HPV-16, 18, 31, 33 ou 45.

12. Composition antitumorale selon l'une des revendications 1 à 11, **caractérisée en ce que** ledit vecteur recombinant comprend en outre les séquences codant pour au moins un composé améliorant l'effet antitumoral de ladite composition.

13. Composition antitumorale selon la revendication 12, **caractérisée en ce que** ledit composé améliorant l'effet antitumoral est un immunostimulateur.

14. Composition antitumorale selon la revendication 13, **caractérisée en ce que** ledit composé immunostimulateur est sélectionné parmi le groupe constitué par l'interleukine-2, I'interleukine-7, I'interleukine-12 et les molécules de co-adhésion B7.1 et B7.2.

15. Composition antitumorale selon l'une des revendications 1 à 14, **caractérisée en ce que** ledit vecteur recombinant est un poxvirus.

16. Composition antitumorale selon la revendication 15, **caractérisée en ce que** ledit poxvirus est un MVA.

17. Composition antitumorale selon l'une des revendications 1 à 16, renfermant un support acceptable d'un point de vue pharmaceutique permettant son administration par injection à l'homme ou à l'animal.

18. Vecteur recombinant comprenant les séquences codant pour un ou plusieurs polypeptide(s) immunogène(s), **caractérisé en ce que** l'un au moins desdits polypeptides présente les caractéristiques définies aux revendications 1 à 17.

19. Particule virale comprenant un vecteur recombinant selon la revendication 18.

20. Composition antitumorale **caractérisée en ce qu'**elle comprend un ou plusieurs polypeptide(s) immunogène(s), **caractérisée en ce que** l'un au moins desdits polypeptides présente les caractéristiques définies aux revendications 1 à 11.

21. Utilisation d'une composition antitumorale selon l'une des revendications 1 à 17 et 20, d'un vecteur recombinant selon la revendication 18 ou d'une particule virale selon la revendication 19, pour la préparation d'un médicament destiné au traitement ou à la prévention d'un cancer ou d'une tumeur.

22. Utilisation selon la revendication 21, pour la préparation d'un médicament destiné au traitement ou à la prévention d'un cancer du col de l'utérus, d'une dysplasie du col de bas grade ou d'une infection à papillomavirus.

## Patentansprüche

1. Antitumorale Zusammensetzung, umfassend mindestens einen rekombinanten Vektor, der Sequenzen einschließt, die ein oder mehrere immunogene(s) Polypeptid(e) kodieren, **dadurch gekennzeichnet, dass** mindestens eines der Polypeptide ein Polypeptid ist, das natürlich eine Nicht-Membranlokalisation aufweist, und durch Insertion einer Membranverankerungssequenz und in dem Fall, in dem dem nativen immunogenen Polypeptid eine solche fehlt, einer Sekretionssequenz auf solche Weise modifiziert ist, dass es eine Membranlokalisation auf der Oberfläche der Zellen aufweist, in denen es exprimiert wird, und **dadurch gekennzeichnet, dass** das immunogene Polypeptid einen Ähnlichkeitsgrad von mehr als 70% mit der Gesamtheit oder einem Teil eines Polypeptids aufweist, das durch eine frühe und/oder späte Region eines Papillomavirus kodiert wird.

2. Antitumorale Zusammensetzung nach Anspruch 1, **dadurch gekennzeichnet, dass** das Polypeptid natürlich eine Zellkernlokalisation aufweist und dass darüber hinaus seine natürliche Zellkernlokalisationssequenz deletiert ist.

3. Antitumorale Zusammensetzung nach einem der Ansprüche 1 und 2, **dadurch gekennzeichnet, dass** die Membranverankerungssequenz und/oder die Sekretionssequenz aus der Gruppe ausgewählt sind, die aus derjenigen des Tollwut-Glycoproteins, des env-Glycoproteins des HIV-Virus und des Proteins F des Masernvirus besteht.

4. Antitumorale Zusammensetzung nach einem der Ansprüche 2 bis 3, **dadurch gekennzeichnet, dass** die Sekretionssequenz am N-terminalen Ende des immunogenen Polypeptids inseriert ist und die Membranverankerungssequenz am C-terminalen Ende desselben inseriert ist.

5. Antitumorale Zusammensetzung nach einem der Ansprüche 1 bis 4, **dadurch gekennzeichnet, dass** das immunogene Polypeptid einen Ähnlichkeitsgrad von mehr als 70% mit einem Polypeptid aufweist, das durch die frühe Region eines Papillomavirus kodiert wird.

6. Antitumorale Zusammensetzung nach Anspruch 5, **dadurch gekennzeichnet, dass** das immunogene Polypeptid ein Polypeptid E6 oder E7 eines Papillomavirus ist.

7. Antitumorale Zusammensetzung nach Anspruch 6, **dadurch gekennzeichnet, dass** das immunogene Polypeptid eine nicht-onkogene Variante des Polypeptids E6 oder E7 eines Papillomavirus ist, das im Bereich der Region mutiert ist, welche an dem Prozess der zellulären Transformation beteiligt ist.

8. Antitumorale Zusammensetzung nach einem der Ansprüche 1 bis 4, **dadurch gekennzeichnet, dass** das immunogene Polypeptid einen Ähnlichkeitsgrad von mehr als 70% mit dem Polypeptid L1 oder L2 eines Papillomavirus aufweist.

9. Antitumorale Zusammensetzung nach einem der Ansprüche 1 bis 8, **dadurch gekennzeichnet, dass** mindestens ein immunogenes Polypeptid einen Ähnlichkeitsgrad von mehr als 70% mit einem frühen Polypeptid und mindestens ein immunogenes Polypeptid einen Ähnlichkeitsgrad von mehr als 70% mit einem späten Polypeptid eines Papillomavirus aufweist.

10. Antitumorale Zusammensetzung nach einem der Ansprüche 1 bis 9, **dadurch gekennzeichnet, dass** mindestens ein immunogenes Polypeptid wie folgt ist:
(1) das immunogene Polypeptid weist eine Sequenz auf, die einen Identitätsgrad von mehr als 70% mit derjenigen aufweist, die in der SEQ ID NO: 1 gezeigt ist, oder mit dieser identisch ist,
(2) das immunogene Polypeptid weist eine Sequenz auf, die einen Identitätsgrad von mehr als 70% mit derjenigen aufweist, die in der SEQ ID NO: 2 gezeigt ist, oder mit dieser identisch ist,
(3) das immunogene Polypeptid weist eine Sequenz auf, die einen Identitätsgrad von mehr als 70% mit derjenigen aufweist, die in der SEQ ID NO: 1 gezeigt ist, oder mit dieser identisch ist, und wobei ein immunogenes Polypeptid eine Sequenz aufweist, die einen Identitätsgrad von mehr als 70% mit derjenigen aufweist, die in der SEQ ID NO: 2 gezeigt ist, oder mit dieser identisch ist,
(4) das immunogene Polypeptid weist eine Sequenz auf, die einen Identitätsgrad von mehr als 70% mit derjenigen aufweist, die in der SEQ ID NO: 1 gezeigt ist, oder mit dieser identisch ist, wobei ein immunogenes Polypeptid das Protein L1 eines Papillomavirus ist und/oder ein immunogenes Polypeptid das Protein L2 eines Papillomavirus ist,
(5) das immunogene Polypeptid weist eine Sequenz auf, die einen Identitätsgrad von mehr als 70% mit derjenigen aufweist, die in der SEQ ID NO: 2 gezeigt ist, oder mit dieser identisch ist, wobei ein immunogenes Polypeptid das Protein L1 eines Papillomavirus ist und/oder ein immunogenes Polypeptid das Protein L2 eines Papillomavirus ist, oder
(6) das immunogene Polypeptid weist eine Sequenz auf, die einen Identitätsgrad von mehr als 70% mit derjenigen aufweist, die in der SEQ ID NO: 1 gezeigt ist, oder mit dieser identisch ist, wobei ein immunogenes Polypeptid eine Sequenz aufweist, die einen Identitätsgrad von mehr als 70% mit derjenigen aufweist, die in der SEQ ID NO: 2 gezeigt ist, oder damit identisch ist, wobei ein immunogenes Polypeptid das Protein L1 eines Papillomavirus ist und/oder ein immunogenes Polypeptid das Protein L2 eines Papillomavirus ist.

11. Antitumorale Zusammensetzung nach einem der Ansprüche 1 bis 10, **dadurch gekennzeichnet, dass** das Papillomavirus ein humanes Papillomavirus ist, wie HPV-16, 18, 31, 33 oder 45.

12. Antitumorale Zusammensetzung nach einem der Ansprüche 1 bis 11, **dadurch gekennzeichnet, dass** der rekombinante Vektor darüber hinaus Sequenzen umfasst, die mindestens eine Verbindung kodieren, welche die antitumorale Wirkung der Zusammensetzung verbessert.

13. Antitumorale Zusammensetzung nach Anspruch 12, **dadurch gekennzeichnet, dass** die Verbindung, welche die antitumorale Wirkung verbessert, ein Immunstimulator ist.

14. Antitumorale Zusammensetzung nach Anspruch 13, **dadurch gekennzeichnet, dass** die Immunstimulator-Verbindung aus der Gruppe ausgewählt ist, die aus Interleukin-2, Interleukin-7, Interleukin-12 und den Coadhäsionsmolekülen B7.1 und B7.2 besteht.

15. Antitumorale Zusammensetzung nach einem der Ansprüche 1 bis 14, **dadurch gekennzeichnet, dass** der rekombinante Vektor ein Poxvirus ist.

16. Antitumorale Zusammensetzung nach Anspruch 15, **dadurch gekennzeichnet, dass** das Poxvirus ein MVA ist.

17. Antitumorale Zusammensetzung nach einem der Ansprüche 1 bis 16, die einen unter einem pharmazeutischen Gesichtspunkt annehmbaren Träger einschließt, welcher deren Verabreichung durch Injektion an einen Menschen oder ein Tier gestattet.

18. Rekombinanter Vektor, umfassend Sequenzen, die ein oder mehrere immunogene(s) Polypeptid(e) kodieren, **dadurch gekennzeichnet, dass** mindestens eines der Polypeptide die Merkmale aufweist, die in den Ansprüchen 1 bis 17 definiert sind.

19. Viruspartikel, umfassend einen rekombinanten Vektor nach Anspruch 18.

20. Antitumorale Zusammensetzung, **dadurch gekennzeichnet, dass** sie ein oder mehrere immunogene(s) Polypeptid(e) umfasst, **dadurch gekennzeichnet, dass** mindestens eines der Polypeptide die Merkmale aufweist, die in den Ansprüchen 1 bis 11 definiert sind.

21. Verwendung einer antitumoralen Zusammensetzung nach einem der Ansprüche 1 bis 17 und 20, eines rekombinanten Vektors nach Anspruch 18 oder eines Viruspartikeis nach Anspruch 19 für die Herstellung eines Medikaments, das zur Behandlung oder Verhütung eines Krebses oder eines Tumors bestimmt ist.

22. Verwendung nach Anspruch 21 für die Herstellung eines Medikaments, das zur Behandlung oder Verhütung eines Krebses des Gebärmutterhalses, einer Dysplasie niedrigen Grades des Halses oder einer Papillomavirus-Infektion bestimmt ist.

## Claims

1. Antitumoral composition comprising at least one recombinant vector containing the sequences encoding one or more immunogenic polypeptides, **characterized in that** at least one of the said polypeptides is a polypeptide naturally exhibiting a nonmembrane localization and is modified by insertion of a membrane anchoring sequence and, should the native immunogenic polypeptide lack it, of a secretory sequence so as to exhibit a membrane localization at the surface of the cells in which it is expressed, and **characterized in that** said immunogenic polypeptide exhibits a degree of similarity greater than 70% with all or part of a polypeptide encoded by an early and/or late region of the papillomavirus.

2. Antitumoral composition according to Claim 1, **characterized in that** the said polypeptide naturally exhibits a nuclear localization and is in addition deleted for its natural nuclear localization sequence.

3. Antitumoral composition according to either of Claims 1 and 2, **characterized in that** the said membrane anchoring sequence and/or the secretory sequence is selected from the group consisting of that of the rabies glycoprotein, of the HIV virus env glycoprotein and of the measles virus F protein.

4. Antitumoral composition according to one of Claims 1 to 3, **characterized in that** the said secretory sequence is inserted at the N-terminal end and the membrane anchoring sequence is inserted at the C-terminal end of the said immunogenic polypeptide.

5. Antitumoral composition according to one of Claims 1 to 4, **characterized in that** the said immunogenic polypeptide exhibits a degree of similarity greater than 70% with the polypeptide encoded by the early region of a papillomavirus.

6. Antitumoral composition according to Claim 5, **characterized in that** the said immunogenic polypeptide is a polypeptide E6 or E7 of a papillomavirus.

7. Antitumoral composition according to Claim 6, **characterized in that** the said immunogenic polypeptide is a nononcogenic variant of the said E6 or E7 polypeptide of a papillomavirus, mutated at the level of the region involved in the cellular transformation process.

8. Antitumoral composition according to one of Claims 1 to 4, **characterized in that** the said immunogenic polypeptide exhibits a degree of similarity greater than 70% with the polypeptide L1 or L2 of a papillomavirus.

9. Antitumoral composition according to one of Claims 1 to 8, **characterized in that** at least one immunogenic polypeptide exhibits a degree of similarity greater than 70% with an early polypeptide and at least one immunogenic polypeptide exhibits a degree of similarity greater than 70% with a late polypeptide of a papillomavirus.

10. Antitumoral composition according to one of Claims 1 to 9, **characterized in that** at least one immunogenic polypeptide is such that:
(1) the said immunogenic polypeptide has a sequence exhibiting a degree of identity greater than 70% or identical to that shown in SEQ ID NO:1,
(2) the said immunogenic polypeptide has a sequence exhibiting a degree of identity greater than 70% or identical to that shown in SEQ ID NO:2,
(3) the said immunogenic polypeptide has a sequence exhibiting a degree of identity greater than 70% or identical to that shown in SEQ ID NO:1 and an immunogenic polypeptide having a sequence exhibiting a degree of identity greater than 70% or identical to that shown in SEQ ID NO:2,
(4) the said immunogenic polypeptide has a sequence exhibiting a degree of identity greater than 70% or identical to that shown in SEQ ID NO:1, an immunogenic polypeptide being the protein L1 of a papillomavirus and/or an immunogenic polypeptide being the protein L2 of a papillomavirus,
(5) the said immunogenic polypeptide has a sequence exhibiting a degree of identity greater than 70% or identical to that shown in SEQ ID NO:2, an immunogenic polypeptide being the protein L1 of a papillomavirus and/or an immunogenic polypeptide being the protein L2 of a papillomavirus, or
(6) the said immunogenic polypeptide has a sequence exhibiting a degree of identity greater than 70% or identical to that shown in SEQ ID NO:1, an immunogenic polypeptide having a sequence exhibiting a degree of identity greater than 70% or identical to that shown in SEQ ID NO:2, an immunogenic polypeptide being the protein L1 of a papillomavirus and/or an immunogenic polypeptide being the protein L2 of a papillomavirus.

11. Antitumoral composition according to one of Claims 1 to 10, **characterized in that** the said papillomavirus is a human papillomavirus such as HPV-16, 18, 31, 33 or 45.

12. Antitumoral composition according to one of Claims 1 to 11, **characterized in that** the said recombinant vector additionally comprises the sequences encoding at least one compound enhancing the antitumour effect of the said composition.

13. Antitumoral composition according to Claim 12, **characterized in that** the said compound enhancing the antitumour effect is an immunostimulator.

14. Antitumoral composition according to Claim 13, **characterized in that** the said immunostimulatory compound is selected from the group consisting of interleukin-2, interleukin-7, interleukin-12 and the coadhesion molecules B7.1 and B7.2.

15. Antitumoral composition according to one of Claims 1 to 14, **characterized in that** the said recombinant vector is a poxvirus.

16. Antitumoral composition according to Claim 15, **characterized in that** the said poxvirus is an MVA.

17. Antitumoral composition according to one of Claims 1 to 16, containing a pharmaceutically acceptable. carrier allowing its administration by injection into humans or into animals.

18. Recombinant vector comprising the sequences encoding one or more immunogenic polypeptides, **characterized in that** at least one of the said polypeptides exhibits the characteristics defined in Claims 1 to 17.

19. Viral particle comprising a recombinant vector according to Claim 18.

20. Antitumoral composition **characterized in that** it comprises one or more immunogenic polypeptides, **characterized in that** at least one of the said polypeptides exhibits the characteristics defined in Claims 1 to 11.

21. Use of an antitumoral composition according to one of Claims 1 to 17 and 20, of a recombinant vector according to Claim 18 or of a viral particle according to Claim 19, for the preparation of a medicament intended for the treatment or prevention of cancer or of a tumour.

22. Use according to Claim 21, for the preparation of a medicament intended for the treatment or prevention of cancer of the cervix, of low-grade cervical dysplasia and of a papillomavirus infection.
